(19) Europäisches Patentamt
European Patent Office
Office européen des brevets

(11) EP 2 588 219 B1

(12) **EUROPEAN PATENT SPECIFICATION**

(45) Date of publication and mention of the grant of the patent:
**29.05.2024 Bulletin 2024/22**

(21) Application number: **11810126.0**

(22) Date of filing: **29.06.2011**

(51) International Patent Classification (IPC):
***B01D 61/00*** *(2006.01)* ***B01D 71/68*** *(2006.01)*
***B01D 35/02*** *(2006.01)* ***B01D 35/28*** *(2006.01)*
***B01D 63/08*** *(2006.01)* ***B01D 69/10*** *(2006.01)*
***G01N 1/40*** *(2006.01)* ***C12Q 1/04*** *(2006.01)*
***C12Q 1/24*** *(2006.01)* ***C12M 1/12*** *(2006.01)*

(52) Cooperative Patent Classification (CPC):
**B01D 69/10; B01D 63/081; B01D 63/087; B01D 69/105; B01D 71/68; C12M 25/02; C12M 25/14; C12Q 1/04; C12Q 1/24;**
B01D 2313/025; B01D 2313/146; B01D 2313/23; B01D 2313/44; G01N 2001/4088

(86) International application number:
**PCT/US2011/042348**

(87) International publication number:
**WO 2012/012172 (26.01.2012 Gazette 2012/04)**

(54) **FILTER PLATE ARTICLE HAVING A WATER-ABSORBENT FILTER ASSEMBLY**

FILTERPLATTENARTIKEL MIT EINER WASSERABSORBIERENDEN FILTERANORDNUNG

FILTRE COMPORTANT UN ENSEMBLE FILTRE ABSORBANT L'EAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.12.2010 US 201061428029 P**
**30.06.2010 US 360489 P**

(43) Date of publication of application:
**08.05.2013 Bulletin 2013/19**

(73) Proprietor: **Neogen Food Safety US HoldCo Corporation**
**Lansing MI 48912 (US)**

(72) Inventors:
- **MILLER, Jesse, D.**
  **Saint Paul, Minnesota 55133-3427 (US)**
- **SWANSON, Steven, P.**
  **Saint Paul, Minnesota 55133-3427 (US)**
- **AYSTA, James, E.**
  **Saint Paul, Minnesota 55133-3427 (US)**
- **WALLER, Clinton, P., Jr.**
  **Saint Paul, Minnesota 55133-3427 (US)**
- **PERCY, Neil**
  **Saint Paul, Minnesota 55133-3427 (US)**
- **LUCAS, Jeffrey, A.**
  **Saint Paul, Minnesota 55133-3427 (US)**
- **SESHADRI, Kannan**
  **Saint Paul, Minnesota 55133-3427 (US)**
- **RASMUSSEN, Jerald, K.**
  **Saint Paul, Minnesota 55133-3427 (US)**
- **WEISS, Douglas, E.**
  **Saint Paul, Minnesota 55133-3427 (US)**

(74) Representative: **Mathys & Squire**
**Theatinerstraße 7**
**80333 München (DE)**

(56) References cited:
**WO-A1-2010/022111** **WO-A1-2010/077619**
**JP-A- 9 206 062** **JP-A- 9 206 062**
**US-A- 5 348 861** **US-A- 5 681 712**
**US-A- 5 681 712** **US-A1- 2001 017 280**
**US-A1- 2003 211 566** **US-A1- 2010 155 323**
**US-B2- 7 534 600**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 588 219 B1

## Description

### Background

When surfaces become contaminated with bacteria, fungi, yeasts, viruses, or other microorganisms, or "microbes," sickness (morbidity) and, sometimes, death (mortality) may result. This is particularly true when surfaces in food processing plants and healthcare facilitates (e.g., hospitals) become contaminated with microorganisms, food processing plants, surfaces (e.g., solid surfaces, equipment surfaces, protective clothing, etc.) may become contaminated. Such contamination may be caused by or transferred to meat or other foods.

In healthcare facilities, microbes may be released onto surfaces (e.g., solid surfaces, equipment surfaces, clothing, etc.) from infected individuals. Once a surface becomes contaminated with microbes, contact with the contaminated surface may easily and readily transfer microbes to other locations, such as another surface, an individual, equipment, or food.

As is well known, microbial contamination and transfer in certain environments may pose significant health risks. For example, the food that leaves a contaminated food processing plant will subsequently be eaten, and may cause sickness and, possibly, death. Microorganisms such as *Listeria monocytogenes, Salmonella enteriditis,* and *Escherichia coli* O157:H7 are of particular concern.

Microbial contamination is of concern in healthcare facilities since some of the patients of such facilities often suffer from infections by pathogenic microbes and, thus, bring the pathogenic microbes into such facilities. Further, many of those who are present in such facilities (e.g., patients) are sick and may be immunologically compromised. These individuals are, thus, at increased risk of becoming sick from infection by the contaminating microbes.

Membrane filtration is a standard step in many methods of analyzing a liquid sample for the presence of microorganisms. Such analyses are commonly performed in the interest of, for example, food safety, water quality, and/or environmental monitoring and/or study. For some liquid samples, the volume to be filtered needs to be large, on the order of a liter or ten liters, in order to detect levels of microorganisms that would escape detection when a smaller portion of the liquid sample is analyzed.

Existing methods for filtration of these liquid samples typically employ a vacuum manifold and a filter membrane. A liquid sample suspected of containing a microorganism is added to a reservoir above the membrane and a vacuum is applied, sucking the liquid through the membrane and into a trap. After all of the liquid has passed through the membrane, the apparatus is disassembled and the filter membrane is removed with forceps, and placed on an agar plate or other culture device to grow and detect colonies of microorganisms.

US 5681712 A discloses a thin film culture plate having a body member including a self-supporting substrate coated on its upper surface with a layer of an adhesive composition and a layer of cold-water soluble powder and having an apertures spacer member attached to the upper surface of the substrate.

JP 9206062 A discloses a culture apparatus for microorganisms comprising a water-proofing substrate sheet, a water-impermeable transparent cover film, a water-retaining layer, and a culture base layer.

WO 2010/077619 A1 discloses a this film culture plate device useful for detecting hemolysin-producing microorganisms is included. The device includes selective agents and/or indicators to differentiate groups or species microorganisms.

US 5348861 discloses a method for detecting microorganisms which produce a low-molecular-weight metabolite having a molecular weight less than or equal to 500 from a substance capable of being metabolized by the microorganisms.

US 7534600 B2 discloses a detection device comprising an absorbent pad, wherein the absorbent pad comprises a growth medium or a detection medium or a combination thereof, a filter on top of and in contact with the absorbent pad, a liquid impermeable template on top of and in contact with the filter, wherein the template comprises a predetermined number of holes through which a liquid sample can flow.

US 2001/017280 A1 discloses a three zone, reinforced, continuous, geometrically symmetrical microporous membrane including a porous support material encapsulated within a middle zone dispersed between an upper and a lower zone, wherein at least one of the three zones has a pore size at least 20 % greater than the pore size of the other two zones.

WO 2010/022111 A1 discloses a thin film culture device for detection of antibiotic-resistant microorganisms including indicators to differentiate staphylococcal from non-staphylococcal microorganisms.

US 2010/155323 A1 discloses a grafted nonwoven substrates for use as filter elements to purify or separate target materials. The grafted nonwoven substrates have average fiber sizes of 0.7 to 15 $\mu$m and a void volume of 50 to 95 %, and a polymer comprising cationic aminoalkyl (meth)acryloyl monomer units grafted to the surface of the nonwoven substrate.

**Summary**

There is a need for a filter plate article that simplifies the sample preparation and test device conditioning required for detecting the presence of microorganisms in an aqueous sample. This need is solved by the filter plate article of claim 1 and the method of testing for the presence of a microorganism in an aqueous sample of claim 14. Preferred embodiments of the invention are defined in the attached dependent claims.

In one aspect, the present disclosure provides a filter plate article for testing the presence of microorganisms in an aqueous sample that includes: a base member, a filter assembly, and a cover sheet. The base member comprises a self-supporting water impervious substrate with first and second generally opposed major surfaces. The filter assembly defines a filter assembly aperture therein, and has a composite filter body mounted across the filter assembly aperture. The composite filter body comprises a microporous membrane having pores with a nominal range of 0.05 μm to 1.2 μm, and a water-absorbent layer in fluid communication with the microporous membrane. The filter assembly is positioned between the cover sheet and the base member, and the water-absorbent layer is positioned between the microporous membrane and the base member. The water-absorbent layer absorbs a weight of water that is at least 0.5 times the weight of the water-absorbent layer prior to absorbing water and wherein the water-absorbent layer comprises a fibrous substrate that is functionalized with a grafted hydrogel polymer. The grafted hydrogel polymer includes grafting monomer units grafted to the surface of the fibrous substrate and wherein the grafting monomer units are selected from the group consisting of ionic grafting monomer units, non-ionic hydrophilic grafting monomer units, and mixtures thereof. The filter assembly is attached to the base member, and wherein the cover sheet is attached (directly or indirectly) to the base member. The cover sheet or the base member comprises a dry coating that includes a water-soluble gelling agent.

In another aspect, the present disclosure provides a method of testing for the presence of a microorganism in an aqueous sample. The method includes providing a filter plate article of the present disclosure, providing an aqueous sample, and passing the aqueous sample firstly through the microporous membrane and secondly through the water-absorbent layer. The water absorbent layer retains an aliquot of water from the aqueous sample. The filter plate article is incubated for an incubation period, and at least a portion of the aliquot of water contacts the microporous membrane throughout the incubation period. An observation is made for the presence or absence of microbial growth.

In another aspect, the present disclosure includes a filtration apparatus for use with a filter plate article, the filtration apparatus including: (a) a filtration assembly including: (i) a filter seat comprising a filter support surface and defining a filter seat inlet, a filter seat outlet; and (ii) a sample head defining a sample head inlet and a sample head outlet; wherein the filter seat is slidably engageable with the sample head to define an insertion gap between the filter support surface and the sample head outlet; and (b) a guide assembly comprising a first guide member and a second guide member, wherein the first guide member and the second guide member are arranged parallel to each other on opposing sides of the filtration assembly to define an insertion path, thereby to guide the filter membrane layer of the filter article into the insertion gap.

In some embodiments, the filtration apparatus includes a toggle clamp arranged to slide the filter seat between an open insertion gap position and a closed insertion gap position. In some embodiments, the filter apparatus includes an actuator arranged to slide the filter seat between an open insertion gap position and a closed insertion gap position, wherein the actuator is one of a pneumatic actuator or a hydraulic actuator. In some embodiments, the filter apparatus sample head inlet includes a fitting to engage a disposable cartridge. In some embodiments the fitting to engage a disposable cartridge is selected from the group consisting of a ball detent, a Luer lock fitting, a bayonet fitting, and a threaded fitting. In some embodiments, the first guide member comprises a first end and a second end, and wherein the second guide member comprises a first end and a second end, and further wherein the first ends of the first and second guide members define an insertion slot arranged to allow a user to insert a filter article having a membrane layer into the filtration apparatus.

In some embodiments, the filtration apparatus further includes a first cover support portion and a second cover support portion, the first and second cover support portions arranged to support a first cover sheet partially affixed to a first major surface of the filter article having a membrane layer. In some further embodiments, the filtration apparatus further includes a third cover support portion and a fourth cover support portion, the third and fourth cover support portions arranged to support a second cover sheet partially affixed to a second major surface of the filter article having a membrane layer.

In some embodiments, the guide assembly further includes a spring member adjacent the first guide portion, thereby to urge the filter membrane layer away from the sample head.

In another aspect, the disclosure includes a parallel filtration apparatus, comprising a plurality of filtration apparatuses of any of the preceding claims. In some embodiments, the filtration apparatuses are operable in parallel.

"Hydrogel" refers to a water-containing gel, having polymer chains that are hydrophilic and will absorb water, yet are insoluble in water. The term hydrogel is used regardless of the state of hydration.

"Microporous" refers to a water-permeable material having pores within a nominal range of 0.05 micrometer to 1.2 micrometer.

"Self-supporting" refers to a material that is able to support its own weight.

"Target microorganism" refers to a particular microorganism (i.e., a species of microorganism) or a particular group of microorganisms (e.g., a particular genus of microorganisms, coliform bacteria, antibiotic-resistant bacteria) to be detected.

"Water-absorbent" refers to material having a capacity to absorb water at a level of at least 20 wt.% relative to a weight of the material.

"Water-absorption capacity" refers to a weight of water absorbed relative to a weight of water-absorbent material.

"Water activity" or "$A_W$" refers to the availability of water and represents the energy status of the water in a system. It is defined as the vapor pressure of water above a sample divided by that of pure water at the same temperature. Pure distilled water has a water activity of exactly one. In general, an $A_W$ value of at least 0.91 is useful for supporting microbial growth.

"Water-soluble" refers to the temperature of water below which a gelling agent can be reconstituted by hydration. In its most convenient usage the gelling agent may be reconstituted at about 20°C, while in some instances a temperature of up to about 90°C may be acceptable, as long as the temperature does not adversely affect viability of target microorganisms.

## Brief Description of the Drawings

- **FIGS. 1A** and **1B** are an exploded side view and a top view, respectively, of an exemplary embodiment of a filter plate article according to the present description;
- **FIG. 2** is a perspective view of an exemplary embodiment of a filter plate article of the present description;
- **FIG. 3A** and **3B** are an exploded side view and a top view, respectively, of an exemplary embodiment of a filter plate article according to the present description, including a spacer layer;
- **FIGS. 4A-4D** are side views of exemplary embodiments of a filter assembly for a detection article of the present description;
- **FIG. 5** is a perspective view of an exemplary embodiment of a filtration apparatus of the present description;
- **FIGS. 6A** and **6B** are each a front view of an exemplary embodiment of a filtration apparatus, showing open (**FIG. 6A**) and closed (**FIG. 6B**) positions;
- **FIGS. 7A** and **7B** are perspective views of an exemplary embodiment filtration apparatus of the present description, showing an exemplary method of inserting an article for detecting microorganisms in an aqueous sample.
- **FIG. 8** is a perspective view of an exemplary embodiment of a multi-filtration apparatus;
- **FIG. 9** is a perspective view of an exemplary embodiment of a filtration apparatus showing optional ramp features;
- **FIG. 10** is a side view of an exemplary embodiment of a filter seat;
- **FIG. 11** is a perspective view of an exemplary embodiment of a filtration apparatus having a disposable cartridge attached.

Like reference numbers in the various figures indicate like elements. Some elements may be present in identical or equivalent multiples; in such cases only one or more representative elements may be designated by a reference number but it will be understood that such reference numbers apply to all such identical elements. Unless otherwise indicated, all figures and drawings in this document are not to scale and are chosen for the purpose of illustrating different embodiments of the disclosure. In particular the dimensions of the various components are depicted in illustrative terms only, and no relationship between the dimensions of the various components should be inferred from the drawings, unless so indicated. Although terms such as "top", bottom", "upper", lower", "under", "over", "front", "back", "outward", "inward", "up" and "down", and "first" and "second" may be used in this disclosure, it should be understood that those terms are used in their relative sense only unless otherwise noted. In particular, in some embodiments certain components may be present in interchangeable and/or identical multiples (e.g., pairs). For these components, the designation of "first" and "second" may apply to the order of use, as noted herein (with it being irrelevant as to which one of the components is selected to be used first).

## Detailed Description

Unless otherwise defined, all technical and scientific terms used herein have the same meaning as commonly understood by one of ordinary skill in the art to which the present disclosure belongs. Methods and materials similar or equivalent to those described herein can be used in the practice of the embodiments of the present disclosure, and exemplified suitable methods and materials are described below. For example, methods may be described that comprise more than two steps. In such methods, not all steps may be required to achieve a defined goal and the present disclosure

envisions the use of isolated steps to achieve these discrete goals. In addition, the materials, methods, and examples are illustrative only and not intended to be limiting.

The articles and methods of the present disclosure may be used for a variety of applications where it is desirable to detect the presence or absence of microorganisms in a sample including, but not limited to, food samples (e.g., raw materials, in-process food materials, finished products), surfaces (e.g., environmental surfaces, food processing surfaces, equipment), water (e.g., surface water, process water), and beverages (e.g., raw milk, pasteurized milk, juice). The samples may consist of solid, semi-solid, gelatinous, or liquid material, alone or in various combinations. The devices of the present description, as well as the described methods, may be used to determine, qualitatively or quantitatively, the presence of one or more microorganisms of interest. The filter plate article of the present description can also be referred to as a microbial detection article.

An exemplary clinical analyte of interest to detect is *Staphylococcus aureus ("S. aureus").* This is a pathogen causing a wide spectrum of infections including: superficial lesions such as small skin abscesses and wound infections; systemic and life threatening conditions such as endocarditis, pneumonia and septicemia; as well as toxinoses such as food poisoning and toxic shock syndrome. Some strains (e.g., Methicillin-Resistant *S. aureus* or MRSA) are resistant to all but a few select antibiotics.

Exemplary analytes of interest to detect in food processing areas are members of the genus *Listeria. Listeria* are classified as gram-positive, rod-shaped bacteria and include the species *Listeria monocytogenes, L. innocua, L. welshimeri, L. seeligeri, L. ivanovii*, and *L. grayi.* Among these, *L. monocytogenes* is responsible for the majority of human listeriosis cases and immunocompromised, pregnant women, elderly, and newborns have increased susceptibility to infection. The most common symptoms of listeriosis are septicemia, meningitis, and miscarriages.

Other microorganisms of particular interest for analytical purposes include prokaryotic and eukaryotic organisms, particularly Gram positive bacteria, Gram negative bacteria, fungi, mycoplasma, and yeast. Particularly relevant organisms include members of the family *Enterobacteriaceae*, or the family *Micrococcaceae* or the genera *Staphylococcus* spp., *Streptococcus* spp., *Pseudomonas* spp., *Enterococcus* spp., *Salmonella* spp., *Legionella* spp., *Shigella* spp. *Yersinia* spp., *Enterobacter* spp., *Escherichia* spp., *Bacillus* spp., *Vibrio* spp., *Clostridium* spp., *Corynebacteria* spp. as well as, *Aspergillus* spp., *Fusarium* spp., and *Candida* spp. Particularly virulent organisms include *Staphylococcus aureus* (including resistant strains such as Methicillin Resistant *Staphylococcus aureus* (MRSA)), *S. epidermidis*, *Streptococcus pneumoniae*, *S. agalactiae, S. pyogenes, Enterococcus faecalis*, Vancomycin Resistant *Enterococcus* (VRE), Vancomycin Resistant *Staphylococcus aureus* (VRSA), Vancomycin Intermediate-resistant *Staphylococcus aureus* (VISA), *Bacillus anthracis*, *Pseudomonas aeruginosa, Escherichia coli, Aspergillus niger, A. fumigatus, A. clavatus*, *Fusarium solani, F. oxysporum, F. chlamydosporum, Vibrio cholera, V. parahemolyticus, Salmonella cholerasuis*, *S. typhi, S. typhimurium, Candida albicans, C. glabrata*, *C. krusei*, *Enterobacter sakazakii*, *Escherichia. coli* 0157, ESBL-containing microorganisms, and multiple drug resistant Gram negative rods (MDR).

FIG. 1A shows an exploded side view of one embodiment of article 100 according to the present disclosure, useful for detecting microorganisms. Article 100 comprises: base member 110 with first and second generally opposed major surfaces 101 and 102; cover sheet 140; and filter assembly 150. Filter assembly 150 has filter assembly aperture 154 defined therein, and composite filter body 155 mounted across filter assembly aperture 154, wherein the composite filter body includes microporous membrane 160 and water-absorbent layer 180. The water-absorbent layer 180 is in fluid communication with microporous membrane 160. Filter assembly 150 is positioned between cover sheet 140 and base member 110, wherein water-absorbent layer 180 is positioned between microporous membrane 160 and base member 110.

Referring now to FIGS. 1A and 1B, in some embodiments of article 100, filter assembly 150 has attachment portion 105 that is coupled to base member 110. In the illustrated embodiment, filter assembly 150 is coupled to base member 110 at attachment portion 105 via a strip of double-sided adhesive tape 130, although a person of ordinary skill in the art will recognize that other coupling techniques (e.g., adhesives, heat-bonding, ultrasonic welding, stitching, or stapling) may be suitable. In some embodiments, filter assembly 150 can be dimensioned to be at least coextensive with the dimensions of base member 110. Coupling filter assembly 150 to base member 110, as described above, advantageously can keep filter assembly 150 properly positioned within article 100.

The cover sheet 140 preferably is coupled (either directly or indirectly) to base member 110. In the illustrated embodiment, cover sheet 140 is coupled to filter assembly 150 via a strip of double-sided adhesive tape 130. It is recognized that other coupling means (e.g., adhesives, heat-bonding, ultrasonic welding, stitching, or stapling) may be suitable to attach cover sheet 140 to filter assembly 150. It is also recognized that cover sheet 140 may be coupled directly to base member 110, provided that filter assembly 150 is disposed between base member 110 and cover sheet 140.

In some further embodiments, filter assembly 150 may comprise tab region 151. The region 151 may extend beyond peripheral boundary 119 of base member 110. Similarly, tab region 151 may extend beyond peripheral boundary 149 of cover sheet 140.

In some other embodiments, cover sheet 140 may comprise tab region 141. Tab region 141 may extend beyond

peripheral boundary 119 of base member 110. Similarly, tab region 141 may extend beyond peripheral boundary 159 of filter assembly 150. It will be understood that any suitable combination of the tab regions may be provided in order to facilitate peeling back cover sheet 140 and base member 110 and exposing filter assembly 150 for a filtering operation.

FIG. 2 shows a perspective view of an embodiment of a filter plate article of the present description. Cover sheet 140 is preferably flexible, and is typically selected to allow cover sheet 140 to be manually peeled back from filter assembly 150, with minimal difficulty. In some embodiments, filter assembly 150 may also be flexible. Base member 110 may also be flexible, although typically base member 110 is selected to be self-supporting and is less flexible than cover sheet 140.

FIGS. 3A and 3B show an embodiment of filter plate article 300 that in general has parts and part numbers that correspond to parts and part numbers in filter plate article 100 (see FIGS. 1A and 1B), differing in that filter assembly 350 further comprises spacer layer 390 defining spacer layer aperture 394 therein, wherein spacer layer 390 is mounted on a major face of filter assembly 350 facing first major surface 301 of base member 310, and wherein spacer layer aperture 394 is in fluid communication with filter assembly aperture 354. When article 100 is mentioned in the present description, it will be understood that article 100 may comprise a spacer layer as described for article 300.

Referring again to FIG. 2, in some exemplary embodiments, base member 110 includes a first substrate 112, optional first adhesive layer 114 adhered to first substrate 112, and optional first dry coating 116. First substrate 112 is "self-supporting" (i.e., is able to support its own weight) and "water impervious" (i.e., does not absorb water at a level of more than 10 wt.%). Some examples of suitable materials for first substrate 112 include polyester, polypropylene, or polystyrene films. Other examples of suitable materials for first substrate 112 include paper or cardboard materials that comprise a waterproof (e.g., polymeric) coating. First substrate 112 may be transparent, translucent, or opaque, depending on whether one wishes to view bacterial colonies through first substrate 112. To facilitate the counting of bacterial colonies, first substrate 112 may have a square grid pattern printed thereon as described, for example, in U.S. Pat. No. 4,565,783 (Hansen et al.). The materials used to construct first substrate 112 should be inert to microorganisms and, preferably, should be compatible with a sterilization process (e.g., ethylene oxide sterilization).

It should be understood that while FIG. 2 shows base member 110 as including optional first dry coating 116, in some typical embodiments the base member may not include a cold-water soluble gelling agent or nutrients, and instead will include a cold-water soluble gelling agent and nutrients in cover sheet 140. In some more typical embodiments, cover sheet 140 includes second substrate 142, optional second adhesive layer 144, and optional second dry coating 146. In some embodiments, optional second dry coating 146 comprises a detection reagent to detect a microorganism. In some embodiments, optional second dry coating 146 comprises a nutrient medium.

Second substrate 142 preferably is transparent or translucent, to permit viewing or imaging of bacterial colonies through second substrate 142, and does not absorb water at a level of more than about 20 wt.%. Non-limiting examples of suitable materials for second substrate 142 include polyester, polypropylene, or polystyrene films. Preferably, cover sheet 140 is flexible. The materials used to construct second substrate 142 should be inert to microorganisms and, preferably, should be compatible with a sterilization process (e.g., ethylene oxide sterilization).

In some embodiments of article 100, base member 110 may include optional first dry coating 116 adhered to first substrate 112 by optional first adhesive layer 114. In some typical embodiments of article 100, cover sheet 140 includes optional second dry coating 146 adhered to second substrate 142 by optional second adhesive layer 144. In typical embodiments, an optional dry coating is included in one of either the cover sheet or the base member, and most typically an optional dry coating is included in only the cover sheet.

When included in article 100, optional first adhesive layer 114 and/or optional second adhesive layer 144 comprises an adhesive material that is typically water-insoluble, should be non-inhibitory to the growth of microorganisms, and should be capable of withstanding the sterilization process, if a sterilization process is used. Preferably, the adhesive material is sufficiently transparent when wet to enable the viewing of bacterial colonies through the respective substrate layer to which it is adhered. In some embodiments, the adhesive material may comprise a pressure-sensitive adhesive, for example, a pressure sensitive iso-octyl acrylate/acrylic acid copolymer adhesive. A suitable pressure sensitive adhesive may contain iso-octyl acrylate in a range from 90 wt.% to 99 wt.% and acrylic acid in a range from 1 wt.% to 10 wt.% of acrylic acid, and a particularly suitable pressure sensitive adhesive has 96 wt.% iso-octyl acrylate and 4 wt.% acrylic acid.

When included in article 100, optional first dry coating 116 and/or optional second dry coating 146 comprises a water-soluble gelling agent (e.g., guar gum, xanthan gum, locust bean gum), as described, for example, in U.S. Patent No. 4,565,783 (Hansen et al.). Optionally, dry coating 116 and/or 146 may further comprise a nutrient medium, a selective agent, a detection reagent, or any combination of two or more of the foregoing. The nutrient medium, selective agent, and/or detection reagent should not interfere with the gelling agent and generally will be chosen based upon the microorganisms to be detected. Examples of suitable nutrients, selective agents and detection reagents to detect microorganisms can be found, for example, in U.S. Pat. Nos. 4,565,783 (Hansen et al.); 5,089,413 (Nelson et al.); 5,364,766 (Mach et al.); 5,443,963 (Lund); 5,462,860 (Mach); 5,601,998 (Mach et al.); 5,635,367 (Lund); and 5,681,712 (Nelson). "Standard Methods Nutrients", as used herein, refers to the nutrient mixture described in Standard Methods for the

Examination of Dairy Products, 14th Edition, American Public Health Association, Washington, D.C. It consists of 5 parts (by weight) peptone, 2.5 parts yeast extract and 1 part dextrose. The amounts of nutrient medium, selective agent and/or detection reagent are prepared such that, when contacted with a predetermined amount of liquid sample, they facilitate the growth and/or detection of a microorganism.

In some embodiments, first dry coating 116 and/or second dry coating 146 (e.g., a dry powder gelling agent; optionally with a dry powdered nutrient, selective agent and/or detection reagent) may be coupled to the respective first substrate 112 and/or second substrate 142 via the adhesive material, as described, for example, in U.S. Pat. No. 4,565,783 (Hansen et al.), or by coating a liquid mixture comprising a gelling agent (optionally including a dry powdered nutrient, selective agent and/or detection reagent) onto the respective substrate or adhesive layer and subsequently drying the mixture, as described, for example, in U.S. Pat. No. 4,565,783 (Hansen et al.).

In some embodiments, first substrate 112 may comprise first dry coating 116 that covers an entire major surface of first substrate 112. In some embodiments, first substrate 112 may comprise first dry coating 116 that covers only a portion of a major surface of first substrate 112.

In some embodiments, second substrate 142 may comprise second dry coating 146 that covers an entire major surface of second substrate 142. In some embodiments, second substrate 142 may comprise second dry coating 146 that covers only a portion of a major surface of second substrate 142.

In typical embodiments, cover sheet 140 of the filter plate article comprises second dry coating 146 that includes the water-soluble gelling agent described *supra*. In some typical embodiments, cover sheet 140 of the filter plate article comprises second dry coating 146 that includes the nutrient medium described *supra*. In some typical embodiments, cover sheet 140 of the filter plate article comprises second dry coating 146 that includes the detection reagent described *infra*. In some typical embodiments, cover sheet 140 of the filter plate article comprises second dry coating 146 that includes the water-soluble gelling agent, the nutrient medium, and the detection reagent, or any combination of these. The inclusion of the water-soluble gelling agent and the nutrient medium in second dry coating 146 of cover sheet 140 is useful for providing for growth of microorganism (if present on microporous membrane 160), and the inclusion of the detection reagent in second dry coating 146 of cover sheet 140 is useful for observing an indication of the presence or absence of microbial growth.

The detection reagent may be detected visually and/or by using an automated detector. The automated detector may comprise an imaging system. The detection reagent may be chromogenic, fluorogenic, or luminogenic. In any embodiment, the detection reagent, when contacted with a predetermined amount of liquid sample reaches a concentration sufficient to detect a target microorganism. Advantageously, the amount of the detection reagent in first dry coating 116 (or second dry coating 146) can be sufficiently high to rapidly detect the presence of a target microorganism even though the high concentration of detection reagent in the liquid sample also inhibits the growth of the target microorganism. In some embodiments, the amount of detection reagent in first dry coating 116 (or second dry coating 146) is sufficient to detect a target microorganism even though it inhibits the growth of the target microorganism. A relatively higher amount of detection reagent in first dry coating 116 (or second dry coating 146) permits more rapid detection of the target microorganisms than a relatively lower amount of detection reagent even though the relatively higher amount may inhibit growth of the microorganisms.

The detection reagent can be a nonspecific indicator of the type of microorganism present or it can be a specific indicator of the type of microorganism present. Non-limiting examples of nonspecific detection reagents include pH indicators (e.g., azobenzene pH indicators (e.g., methyl red), sulfonphthalein pH indicators (e.g., bromocresol purple, chlorophenol red, bromthymol blue, bromcresol blue), anthroquinone pH indicators (e.g., alizarin red s monohydrate 3,4-dihydroxy-9,10-dioxo-2-anthracensulfonic acid, sodium salt)), and redox indicators (e.g., triphenyltetrazolium chloride (TTC), 3-[4,5-dimethylthiazol-2-yl]-2,5-diphenyltetrazolium bromide (MTT), 2,3-bis[2-methoxy-4-nitro-5-sulfopheny]-2H-tetrazolium-5-carboxyanilide inner salt (XTT), Nitro Blue Tetrazolium).

The detection reagent can be a specific indicator of the type of microorganism present. Specific detection reagents include, for example, substrates for enzymes (e.g., glycosidases, proteases, aminopeptidases, and phosphatases, esterases) that are associated with certain microorganisms or groups of microorganisms.

The detection reagent may be capable of forming a colored precipitate. A variety of dyes are known that could be incorporated into the methods and devices of the present disclosure, including indolyl-containing dyes, for example 5-bromo-4-chloroindolyl phosphate or disodium salts of that compound, 5-bromo-4-chloroindolyl pyranoside or disodium salts of that compound, 5-bromo-4-chloro-3 indolyl-β-D-glucuronic acid, 5-bromo-4-chloro-3-indoxyl-β-D-galactoside, 5-bromo-4-chloro-3-indolyl- β -D-glucopyranoside, 6-chloro-3-indolylphosphate, and 5-bromo-6-chloro-3-indolylphosphate.

Preferably, the colored precipitate is blue. Substrates that create a blue colored precipitate include, for example, 5-bromo-4-chloro-3-indoxyl-N-acetyl-β-D-galactosaminide, 5-bromo-4-chloro-3-indoxyl-N-acetyl-β-D-glucosaminide, 5-bromo-4-chloro-3-indoxyl-β-D-cellobioside, 5-bromo-4-chloro-3-β-D-fucopyranoside, 5-bromo-4-chloro-3-indoxyl-α-D-galactopyranoside, 5-bromo-4-chloro-3-indoxyl-β-galactopyranoside, 5-bromo-4-chloro-3-indoxyl-α-D-glucopyranoside, 5-bromo-4-chloro-3-indoxyl-β-D-glucopyranoside, 5-bromo-4-chloro-3-indoxyl-β-D-glucuronic acid, cyclohexylammonium salt, 5-bromo-4-chloro-3-indoxyl-β-D-glucuronic acid, sodium salt, and 5-bromo-4-chloro-3-indoxyl-β-D-xylopyranoside.

The dyes can serve as substrates for particular enzymes present within certain types of bacteria. Blue-precipitate producing dyes that are substrates for esterases include, for example, 5-bromo-4-chloro-3-indoxyl butyrate, 5-bromo-4-chloro-3-indoxyl caprylate, and 5-bromo-4-chloro-3-indoxyl palmitate. Substrates for phosphatases include, but are not limited to, 5-bromo-4-chloro-3-indoxyl phosphate di(2-amino-2-methyl-1,3-propanediol) salt, 5-bromo-4-chloro-3-indoxyl phosphate disodium salt, 5-bromo-4-chloro-3-indoxyl phosphate and p-toluidine salt, 5-bromo-4-chloro-3-indoxyl phosphate and the potassium salt.

Chromogenic dyes that are substrates for glycosidases include, for example, 3-indoxyl-β-D-galactopyranoside, 3-indoxyl-β-D-glucopyranoside, 3-indoxyl-β-D-glucuronic acid cyclohexylammonium salt, and 3-indoxyl-β-D-glucuronic acid sodium salt. Other chromogenic substrates for phophatases include, for example, 3-indoxyl phosphate di(2-amino-2-methyl-1,3-propanediol) salt, 3-indoxyl phosphate disodium salt, and 3-indoxyl phosphate p-toluidine salt. Substrates for sulfatases include, for example, 3-indoxyl sulfate potassium salt.

Precipitable dyes that produce a magenta color for glycosidases, esterases, phosphatases and sulfatases include, for example, 5-bromo-6-chloro-3-indoxyl-N-acetyl-β-D-glucosaminide, 5-bromo-6-chloro-3-indoxyl-β-D-galactopyranoside, 5-bromo-6-chloro-3-indoxyl-β-D-galactopyranoside, 5-bromo-6-chloro-3-indoxyl-β-D-glucopyranoside, 5-bromo-6-chloro-3-indoxyl-β-glucuronic acid, cyclohexylammonium salt as substrates for glycosidases; 5-bromo-6-chloro-3-indoxyl butyrate, 5-bromo-6-chloro-3-indoxyl caprylate, and 5-bromo-6-chloro-3-indoxyl palmitate serve as substrates for esterases; 5-bromo-6-chloro-3-indoxyl phosphate, p-toluidine salt for phosphatases and 5-bromo-6-chloro-3-indoxyl sulfate, potassium salt serve as substrates for sulfatases.

Precipitable dyes that produce a salmon color for glycosidases, esterases and phosphatases include, for example, 6-chloro-3-indoxyl-β-galactopyranoside, 6-chloro-3-indoxyl- β -D-glucopyranoside, and 6-chloro-3-indoxyl-β-D-glucuronic acid, cyclohexylammonium salt for glycosidases; 6-chloro-3-indoxyl butyrate, 6-chloro-3-indoxyl caprylate, and 6-chloro-3-indoxyl palmitate for esterases; and, 6-chloro-3-indoxyl phosphate, p-toluidine salt for phosphatases.

Chromogenic substrates that produce a purple color include 5-iodo-3-indoxyl-β-D-galactopyranoside and chromogenic substrates that produce a green color include N-methyl-indoxyl-β-D-galactopyranoside.

Other precipitable dyes include 4,6-dichloro-N-acetylindol-3-ol, 6-chloroindolyl-β-D-galactoside pentaacetate, 6-chloroindolyl-β-D-galactoside, 6-chloroindoxy-1,3-diacetate, 5-chloro-2-carboxyphenylglycine sodium salt, 4-chloroanthranilic acid, methyl[6-chloro-N-acetylindol-3-yl-(2,3,4-tri-O-acetyl-β-D-glucopyran oside)]uronate, 6-chloroindolyl-β-D-glucopyranoside uronate monocyclohexylammonium salt, chloroindigos (including those chloroindigos reported by Sadler et al., J. Am Chem. Soc. 78:1251-1255, 1956), as well as 4,6-dichloroindolyl-β-D-glucuronide, 6,7-dichloroindolyl-β-D-glucuronide, 6,7-dichloroindolyl- β -D-glucuronide, 4,6,7-trichloroindolyl-β-D-glucuronide, 4,6-dichloroindolyl- β -D-galactoside, 6,7-dichloroindolyl-β-D-galactoside, and 4,6,7-trichloroindolyl- β -D-galactoside.

Suitable detection reagents further include non-precipitating indicator dyes (i.e., water-soluble dyes that are capable of diffusing in a hydrogel). Non-precipitating indicator dyes include pH indicators (e.g., azobenzene pH indicators, sulfonphthalein pH indicators, and anthroquinone pH indicators, as described herein. Non-precipitating indicator dyes also include chromogenic enzyme substrates that react with an enzyme to release a water-soluble dye (e.g., p-nitrophenyl phosphate or p-nitrophenyl-β-D-glucoside, which each can be hydrolyzed to p-nitrophenol), and fluorogenic enzyme substrates, which can react with an enzyme to release a water-soluble fluorescent dye (e.g., 4-methylumbelliferyl phosphate or 4-methylumbelliferyl-β-D-glucoside, which each can be hydrolyzed to 4-methylumbelliferone).

In the embodiment of article 100 shown in FIGS. 1A and 1B, filter assembly 150 is shown to have filter support layer 158 having filter assembly aperture 154 defined therein, and composite filter body 155 mounted across the filter assembly aperture. Filter support layer 158 is configured to composite filter body 155. Filter support layer 158 preferably is self-supporting and does not absorb water, or absorbs water at a level of less than 10 wt.%. Non-limiting examples of suitable materials for filter support layer 158 include polyester, polypropylene, or polystyrene films. Other suitable materials include paper or cardboard materials that comprise a waterproof (e.g., polymeric) coating. Filter support layer 158 may be transparent, translucent, or opaque. Optionally, filter support layer 158 may comprise tab region 151 that extends beyond a peripheral boundary of base member 110 and/or cover sheet 140.

Filter support layer 158 further comprises filter assembly aperture 154 defined therein that permits fluid communication from base member 110 through filter assembly 150 to cover sheet 140. Filter assembly aperture 154 can be any shape, such as a circle, a square, a rectangle, a hexagon, an octagon, an oval, or an irregular shape, for example.

Composite filter body 155 includes microporous membrane 160 and water-absorbent layer 180, and in FIG. 1A microporous membrane 160 and water-absorbent layer 180 are shown as being mounted on opposed major faces of filter support layer 158, and defining gap 170 therebetween. Water-absorbent layer 180 is in fluid communication with microporous membrane 160. It will be understood that in some embodiments, at least a portion of microporous membrane 160 may contact at least a portion of water-absorbent layer 180, and that in some embodiments gap 170 may not be continuous across filter assembly aperture 154, and that in some embodiments, microporous membrane 160 and the water-absorbent layer may contact each other throughout an area defined by filter assembly aperture 154.

Microporous membrane 160 should be positioned such that, during use, at least a portion of microporous membrane 160 overlays filter assembly aperture 154. Preferably, during use, microporous membrane 160 is positioned such that microporous membrane 160 overlays filter assembly aperture 154. In some embodiments, the area of microporous membrane 160 is larger than the area of filter assembly aperture 154, and microporous membrane 160 extends beyond the perimeter of filter assembly aperture 154.

In the embodiment of article 300 shown in FIGS. 3A and 3B, filter assembly 350 further comprises spacer layer 390 defining spacer layer aperture 394 therein, wherein spacer layer 390 is mounted on a major face of filter support layer 358 facing first major surface 301 of base member 310, and wherein spacer layer aperture 394 is in fluid communication with filter assembly aperture 354. Spacer layer 390 should be constructed from a water-insoluble material. The walls of the aperture spacer layer, together with filter support layer 358, form a liquid sample well of predetermined size and shape to confine a volume of liquid sample (e.g., an aqueous sample) within article 300. Spacer layer 390 should be thick enough and spacer layer aperture 394 large enough to form a well of the desired volume (e.g., 1 milliliter, 2 milliliters, 3 milliliters, 5 milliliters, or more). Closed cell polyethylene foam or polystyrene foam are suitable materials for spacer layer 390, but any material which is hydrophobic (non-wetting), inert to microorganisms, and, preferably, capable of withstanding a sterilization process may be used. Spacer layer 390 can be coupled to filter support layer 358, for example, by an adhesive, as described for example in U.S. Pat. No. 4,565,783 (Hansen et al.).

FIGS. 4A-4D show several alternative embodiments for positioning microporous membranes 460A-D and water-absorbent layers 480A-D across filter assembly apertures 454A-D. FIG. 4A shows filter assembly 450A having features essentially the same as filter assembly 150 in FIGS. 1A and 1B, but including optional scrim 475A positioned within composite filter body 455A, between microporous membrane 460A and water-absorbent layer 480A. Optional scrim 475A may be useful to provide reinforcement for composite filter body 455A, while maintaining fluid communication of water-absorbent layer 480A with microporous membrane 460A. Scrim materials are described along with a more detailed description of the water-absorbent layer, below.

FIG. 4B shows an embodiment of filter assembly 450B having composite filter body 455B mounted across filter assembly aperture 454B, composite filter body 455B contacting only one major surface of filter support layer 458B, and wherein water-absorbent layer 480B is positioned between microporous membrane 460B and the major surface of filter support layer 458B.

FIG. 4C shows an embodiment of filter assembly 450C having composite filter body 455C mounted across filter assembly aperture 454C, composite filter body 455C contacting only one major surface of filter support layer 458C, and wherein microporous membrane 460C is positioned between water-absorbent layer 480C and the major surface of filter support layer 458C.

FIG. 4D shows an embodiment of filter assembly 450D, wherein a perimeter of composite filter body 455D fits within filter assembly aperture 454D.

In any of the embodiments in FIGS. 1A and 4A-4D, at least one gasket may be provided to mount composite filter body 155 (or 455A-D) onto filter support layer 158 (or 458A-D), as is shown, for example, in FIG. 4D, where at least one gasket 490D may be provided to secure composite filter body 455D to filter support layer 458D. The gasket may be of any suitable material, for example, biaxially-oriented polypropylene (BOPP), and may further comprise a layer of an adhesive to affix to both composite filter body 155 (or 455A-D) and the filter support layer. In the embodiment shown in FIG. 1A, for example, a first gasket (not shown in FIG. 1A) may be provided to mount microporous membrane 160 onto a first major surface of filter support layer 158, and a second gasket (not shown in FIG. 1A) may be provided to mount water-absorbent layer 180 onto a second major surface of filter support layer 158.

A person of ordinary skill in the art will recognize that additional techniques for mounting the components of composite filter body 155 onto filter support layer 158 may be suitable, including, for example, adhesives, heat-bonding, ultrasonic welding, or combinations of these.

Microporous membrane 160 (or any of microporous membranes 460A-D) can comprise any one of a variety of water-permeable microporous membrane materials known in the art including, for example, cellulosic membranes (e.g., cellulose acetate, mixed cellulose esters, and nitrocellulose), nylon, polycarbonate, polyethersulfone, polyester, polyvinylidene fluoride, ceramic, a derivative of any of the foregoing, and a combination of any two or more of the foregoing. The nominal pore size of microporous membrane 160 is in the range of 0.05 micrometer to 1.2 micrometer, typically selected according to microorganisms to be detected. For example, a nominal pore size in a range of 0.05 micrometer to 1.2 micrometer includes any of 0.05 micrometer, 0.1 micrometer, 0.2 micrometer, 0.45 micrometer, 0.8 micrometer, or 1.2 micrometer. In some embodiments a nominal pore size in a range of 0.2 micrometer to 0.45 micrometer, can be used to detect bacteria. In some embodiments, a nominal pore size in a range of 0.05 micrometer to 3 micrometers (including any of 0.45 micrometer, 0.8 micrometer, 1.2 micrometer, or 3 micrometers), or in some embodiments a range of 0.45 micrometer to 1.2 micrometer, can be used to detect yeast and/or molds. In any embodiment, the microporous membrane should be configured to collect microorganisms from an aqueous sample filtered through the filter assembly. Water permeability test measurements may include, for example, those described in ASTM F2298-03.

Examples of useful commercial microporous membranes include cast nylon microporous membranes available

from 3M Purification, Inc. (Meriden, CT) under the trade designations "LIFEASSURE" and "STERASSURE". Useful microporous membranes are described, for example, in U.S. Pat. Nos. 6,413,070 (Meyering et al.), 6,513,666 (Meyering et al.), 6,776,940 (Meyering et al.), 6,056,529 (Meyering et al.), 6,264,044 (Meyering et al.), 5,006,247 (Dennison et al.), U.S. 3,876,738 (Marinaccio et al.), U.S. 4,707,265 (Barnes et al.), and U.S. 4,473,474 (Ostreicher et al.). Useful graft polymer functionalized microporous membranes are described, for example, in U.S. Published Pat. App. No. 2010/0261801 (Weiss et al.), published October 14, 2010.

In some embodiments of article 100, the water-absorbent layer 180 in the composite filter body 155 comprises a fibrous substrate that is functionalized with a grafted hydrogel polymer. In some embodiments, the fibrous substrate is a nonwoven substrate. As used herein, the term "nonwoven" means a textile structure produced by bonding or interlocking of fibers, or both, accomplished by mechanical, chemical, thermal, or solvent means and combinations in accordance with ASTM D123-09e1.

In some embodiments, water-absorbent layer 180 can comprise a fibrous substrate that is a woven substrate functionalized with a grafted hydrogel polymer. "Woven substrate" as used herein can include cloths or fabrics. As used herein, "woven" means a structure produced when at least two sets of strands are interlaced, usually at right angles to each other, according to a predetermined pattern of interlacing, and such that at least one set is parallel to the axis along the lengthwise direction of the fabric, in accordance with ASTM D123-09e1. These substrates are typically made from natural fibers (e.g., cotton or wool), synthetic fibers (e.g., nylon, polyester, acrylonitrile), or blends thereof. While the grafting chemistry described herein is primarily in terms of nonwoven substrates, it will be understood that the grafting chemistry can also be applied to woven substrates for use as the water-absorbent layer 180 in filter plate article 100.

The fibrous substrate may have an average fiber size in a range from 0.7 micrometer to 15 micrometers, and a void volume in a range from 50% to 95%.

The grafted hydrogel polymer includes grafting monomer units grafted to the surface of the fibrous substrate, wherein the grafting monomer units are selected from the group consisting of ionic grafting monomer units, non-ionic hydrophilic monomer units, and mixtures thereof. In some embodiments, the grafted hydrogel polymer is a cationic hydrogel polymer comprising cationic aminoalkyl(meth)acryloyl monomer units (described *infra*), such as (3-[(methacryloylamino)propyl]trimethylammonium chloride) (MAPTAC), grafted to the surface of the fibrous substrate. In some other embodiments, the grafted hydrogel polymer is an anionic hydrogel polymer, comprising anionic monomer units (described *infra*), such as 2-acrylamido-2-methylpropanesulfonic acid (AMPS), grafted to the surface of the fibrous substrate. In some other embodiments, the grafted hydrogel polymer is a non-ionic hydrogel polymer, comprising non-ionic hydrophilic monomer units (described *infra*), such as dimethylacrylamide (DMA) or 2-hydroxyethyl(meth)acrylate (HEMA), grafted to the surface of the fibrous substrate. In some embodiments the grafted hydrogel polymer is a combination of cationic aminoalkyl(meth)acryloyl monomer units and anionic monomer units grafted to the surface of the fibrous substrate. In some embodiments, the grafted hydrogel polymer is a combination of ionic (cationic and/or anionic) and non-ionic hydrophilic monomers grafted to the surface of the fibrous substrate.

In some embodiments, the fibrous substrate includes a nonwoven web which may include nonwoven webs manufactured by any of the commonly known processes for producing nonwoven webs. As used herein, the term "nonwoven web" refers to a fabric that has a structure of individual fibers or filaments which are randomly and/or unidirectionally interlaid in a mat-like fashion.

For example, the nonwoven web can be made by carded, air laid, wet laid, spunlaced, spunbonding, electrospinning or melt-blowing techniques, such as melt-spun or melt-blown, or combinations thereof. Spunbonded fibers are typically small diameter fibers that are formed by extruding molten thermoplastic polymer as filaments from a plurality of fine, usually circular capillaries of a spinneret with the diameter of the extruded fibers being rapidly reduced. Meltblown fibers are typically formed by extruding the molten thermoplastic material through a plurality of fine, usually circular, die capillaries as molten threads or filaments into a high velocity, usually heated gas (e.g., air) stream which attenuates the filaments of molten thermoplastic material to reduce their diameter. Thereafter, the meltblown fibers are carried by the high velocity gas stream and are deposited on a collecting surface to from a web of randomly disbursed meltblown fibers. Any of the nonwoven webs may be made from a single type of fiber or two or more fibers that differ in the type of thermoplastic polymer and/or thickness.

Staple fibers may also be present in the web. The presence of staple fibers generally provides a loftier, less dense web than a web of only melt blown microfibers. Preferably, no more than 20 wt.% (weight percent) staple fibers are present, more preferably no more than 10 wt.%. Such webs containing staple fiber are disclosed in U.S. Pat. No. 4,118,531 (Hauser).

The fibrous substrate may optionally further include, for example, one or more layers of scrim. For example, either or both of the major surfaces may optionally comprise a scrim layer. The scrim, which is typically a woven or nonwoven reinforcement made from fibers, is included to provide strength to the nonwoven substrate. Suitable scrim materials include, but are not limited to, nylon, polyester, fiberglass, and the like. The average thickness of the scrim can vary. Typically, the average thickness of the scrim is in a range from 25 micrometers to 100 micrometers, or in some embodiments a range from 25 micrometers to 50 micrometers. The layer of the scrim may optionally be bonded to the

nonwoven substrate. A variety of adhesive materials can be used to bond the scrim to the polymeric material. Alternatively, the scrim may be heat-bonded to the nonwoven.

The microfibers of the nonwoven substrate typically have an effective fiber diameter of from 0.5 micrometer to 15 micrometers, preferably from 1 micrometer to 6 micrometers, as calculated according to the method set forth in Davies, C. N., "The Separation of Airborne Dust and Particles," Institution of Mechanical Engineers, London, Proceedings 1B, 1952. The nonwoven substrate preferably has a basis weight in the range of 10 to 400 g/m$^2$ (grams per meter squared), more preferably 10 to 100 g/m$^2$. The average thickness of the nonwoven substrate is preferably 0.1 millimeter to 10 millimeters, more preferably 0.25 millimeter to 5 millimeters for the non-functionalized, uncalendered substrate. The minimum tensile strength of the nonwoven web is about 4 Newtons. It is generally recognized that the tensile strength of nonwovens is lower in the machine direction than in the cross-web direction due to better fiber bonding and entanglement in the latter.

Nonwoven web loft is measured by solidity, a parameter that defines the solids fraction in a volume of web. Lower solidity values are indicative of greater web loft. Useful nonwoven substrates have a solidity of less than 20%, preferably less than 15%. Solidity is a unitless fraction typically represented by $\alpha$:

$$\alpha = m_f \div \rho_f \times L_{nonwoven}$$

where $m_f$ is the fiber mass per sample surface area, which $\rho_f$ is the fiber density; and

$$L_{nonwoven}$$

is the nonwoven thickness. Solidity is used herein to refer to the nonwoven substrate itself and not to the functionalized nonwoven. When a nonwoven substrate contains mixtures of two or more kinds of fibers, the individual solidities are determined for each kind of fiber using the same $L_{nonwoven}$ and these individual solidities are added together to obtain the web's solidity, $\alpha$.

In some embodiments, the nonwoven substrate has a mean pore size of 1 micrometer to 40 micrometers, or in some embodiments even 2 micrometers to 20 micrometers. Mean pore size may be measured according to ASTM F316-03 Standard Test Methods for Pore Size Characteristics of Membrane Filters by Bubble Point and Mean Flow Pore Test Method B using a test fluid available from DuPont (Wilmington, DE) under the trade designation "FREON TF".

The nonwoven substrate may be formed from any suitable thermoplastic polymeric material. Suitable polymeric materials include, but are not limited to, polyolefins, poly(isoprenes), poly(butadienes), fluorinated polymers, chlorinated polymers, polyamides, polyimides, polyethers, poly(ether sulfones), poly(sulfones), poly(vinyl acetates), copolymers of vinyl acetate, such as poly(ethylene) -co-poly(vinyl alcohol),, poly(phosphazenes), poly(vinyl esters), poly(vinyl ethers), poly(vinyl alcohols), and poly(carbonates).

Suitable polyolefins for the nonwoven substrate include, but are not limited to, poly(ethylene), poly(propylene), poly(1-butene), copolymers of ethylene and propylene, alpha olefin copolymers (such as copolymers of ethylene or propylene with 1-butene, 1-hexene, 1-octene, and 1-decene), poly(ethylene-co-1-butene) and poly(ethylene-co-1-butene-co-1 -hexene).

Suitable fluorinated polymers for the nonwoven substrate include, but are not limited to, poly(vinyl fluoride), poly(vinylidene fluoride), copolymers of vinylidene fluoride (such as poly(vinylidene fluoride-co-hexafluoropropylene), and copolymers of chlorotrifluoroethylene (such as poly(ethylene-co-chlorotrifluoroethylene).

Suitable polyamides for the nonwoven substrate include, but are not limited to, nylon 6, nylon 6,6, nylon 6,12 poly(iminoadipoyliminohexamethylene), poly(iminoadipoyliminodecamethylene), and polycaprolactam. Suitable polyimides include poly(pyromellitimide).

Suitable poly(ether sulfones) for the nonwoven substrate include, but are not limited to, poly(diphenylether sulfone) and poly(diphenylsulfone-*co*-diphenylene oxide sulfone).

Suitable copolymers of vinyl acetate for the nonwoven substrate include, but are not limited to, poly(ethylene-*co*-vinyl acetate) and such copolymers in which at least some of the acetate groups have been hydrolyzed to afford various poly(vinyl alcohols) including, poly(ethylene-*co*-vinyl alcohol).

In some embodiments, the polymers for the nonwoven substrate are inherently hydrophilic and are readily grafted by ionizing radiation, such as e-beam or gamma radiation. Useful polymers include polyamides and ethylene vinyl alcohol polymers and copolymers. Nylon nonwoven substrates having 1 micrometer or smaller effective fiber diameters may be chosen from those described, for example, in U.S. Pat. Nos. 7,170,739 (Arora et al.), 7,112,389 (Arora et al.), 7,235,122, (Bryner et al.), and U.S. Published Pat. App. No. 2004/0116026. Useful nylon nonwoven substrates having 1 $\mu$m or smaller effective fiber diameters include, for example, hybrid membrane technology membranes available from DuPont (Wilmington, DE) under the trade designations "HMT 16434" and "HMT 16435".

Further details on the manufacturing method of nonwoven substrates of this description may be found, for example, in Wente, Superfine Thermoplastic Fibers, 48 Indus. Eng. Chem. 1342 (1956), or in Wente et al., Manufacture Of Superfine Organic Fibers, (Naval Research Laboratories Report No. 4364, 1954). Useful methods of preparing the nonwoven substrates are described, for example, in U.S. RE39,399 (Allen), and U.S. Pat. Nos. 3,849,241 (Butin et al.), 7,374,416 (Cook et al.), 4,936,934 (Buehning), and 6,230,776 (Choi).

In typical embodiments, the grafted hydrogel polymer comprises polymer chains (tendrils) that are initiated from, and supported by, the nonwoven substrate, the polymer tendrils extending into the interstitial spaces of the nonwoven substrate. In some embodiments of the nonwoven substrate, the grafted hydrogel polymer chains have pendent groups that are cationic. In some other embodiments of the nonwoven substrate, the grafted hydrogel polymer chains have pendent groups that are anionic. In some embodiments of the nonwoven substrate, the grafted hydrogel polymer chains have pendant groups that are non-ionic hydrophilic functional groups (described *infra*). In the presence of pure water the hydrogel reaches a state of maximum hydration and volume. As the polymer tendrils may be free to move independently, the nonwoven substrate having the grafted hydrogel polymer may have a large flow response to very low quantities of stimulus.

Depending on the degree of substitution of the nonwoven substrate and the weight of hydrogel polymer grafted to the surface thereof, the grafted hydrogel polymer can completely fill the interstitial spaces of the nonwoven substrate thereby providing a barrier which effectively blocks the flow of pure water through the functionalized nonwoven article. The grafted hydrogel polymer, which may be considered to exist as a hydrogel network, can reversibly expand in response to a very small amount of a "trigger" such as a salt, a buffer, an organic solvent, a temperature, or a pH, consequently contracting and thereby allowing for higher flux at lower pressure through the hydrogel network. In the absence of such a "trigger", the fully expanded hydrogel network can offer resistance to water flux.

In the state of maximum hydration, the grafted hydrogel polymer is constrained only by the nonwoven substrate, most significantly in the x and y axes (coplanar with the nonwoven substrate) and less so in the z axis, normal to the plane of the nonwoven substrate. The grafted hydrogel polymer is less constrained on the z axis. The grafted hydrogel polymer may swell up to 800 percent or more on the z axis, but the x and y axes desirably swell less than 100%, more preferably less than 50%, constrained by the nonwoven substrate.

In the preparation of melt-blown nonwoven webs conditions can be adjusted to maximize the resiliency in the z direction (normal to the plane of the nonwoven by (a) adjusting the die and collector for proper fiber lay-down (b) adjusting melt temp and air temp to prevent fibers from over-fusing and forming fiber-fiber bonds, (c) minimize asymmetry caused by the collector being too close in proximity to the die. It is preferred that nonwoven fibers are below the polymer melt temperature before impinging on the collector to reduce the degree of fiber-fiber links. Desirably, the nonwoven substrate may expand maximally in "z" direction (normal to the plane of the nonwoven) to allow for expansion of the grafted hydrogel polymer.

The grafted hydrogel polymer reversibly contracts and allows water to flow (flux) through the resulting interstices in the presence of dissolved species, such as neutral compounds, salts, and buffers. Without being bound by theory, it is believed that when pendant groups in the grafted hydrogel polymer include ionized groups, the dissolved species such as dissolved ions more effectively charge-couple to the ionized groups in the pendant groups in the graft hydrogel polymer so that the electrostatic repulsion between the ionized groups are reduced and the hydrogel constricts or collapses. When the ionized groups in the pendant groups in the grafted hydrogel polymer are anionic, the addition of negatively charged ions dissolved in water results in reversible contraction of the hydrogel, potentially by reducing electrostatic repulsion between the negatively charged anionic groups. Similarly, when the ionized groups in the pendant groups in the graft polymer are cationic, the addition of positively charged ions dissolved in water results in reversible contraction of the hydrogel, potentially by reducing electrostatic repulsion between the positively charged cationic groups (e.g., quaternary ammonium groups). Alternatively the dissolved species may displace the hydration sphere of the water (and possible solvent) molecules with the result that the hydrogel collapses around the nonwoven substrate. Therefore the article exhibits a stimulus-response hydrogel ("responsive hydrogel") that is discontinuous in nature - able to reversibly open and close the pores or interstices of the hydrogel.

In some embodiments, the nonwoven substrate has an ionic grafted hydrogel polymer comprising anionic monomer units grafted to the surface of the nonwoven substrate. The hydrogel polymer is grafted to the surface(s) of the nonwoven substrate by ionization initiated polymerization of "ionic grafting monomers" that are anionic (i.e., negatively charged). Examples of useful nonwoven substrates comprising anionic monomer units grafted to the surface of the nonwoven substrate, and methods of grafting, are described, for example, in International Patent Application No. PCT/US2010/038488 (Waller et al.).

The anionic monomer has at least one ethylenically unsaturated group capable of undergoing free radical polymerization, and an additional anionic functional group. In some embodiments, the ethylenically unsaturated group is a (meth)acryloyl group or a vinyl group. The anionic monomer can be a weak acid, a strong acid, a salt of a weak acid, a salt of a strong acid, or combinations thereof. That is, the anionic monomer can be in a neutral state but capable of being charged if the pH is adjusted. When the pH is suitably adjusted, the resulting graft materials have negatively

charged groups capable of interacting with positively charged materials (i.e., cations). If the anionic monomer includes a salt of a weak acid or a salt of a strong acid, the counter ions of these salts can be, but are not limited to, alkali metals, alkaline earth metals, ammonium ions, or tetraalkylammonium ions.

The anionic monomers may have the general Formula (I):

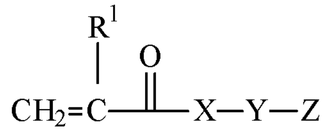

(I)

where

$R^1$ is H or $CH_3$;
X is -O- or $-NR^1-$,
Y is a straight or branched chain alkylene, generally from 1 to 10 carbon atoms; and
Z is an anionic group, which may be selected from sulphonic acids groups, phosphonic acid groups, and carboxylic acid groups, and salts thereof.

Some exemplary anionic monomers include (meth)acrylamidosulfonic acids of Formula (II) or salts thereof:

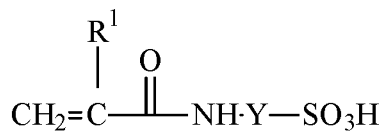

(II)

where $R^1$ is H or $CH_3$, and Y is a straight or branched alkylene having 1 to 10 carbon atoms. Exemplary anionic monomers according to Formula (II) include, but are not limited to, N-acrylamidomethanesulfonic acid, 2-acrylamidoethanesulfonic acid, 2-acrylamido-2-methylpropanesulfonic acid (AMPS), and 2-methacrylamido-2-methylpropanesulfonic acid. Salts of these acidic monomers can also be used, examples being (3-sulfopropyl)-methacrylic acid potassium salt and 2-(methacryloyloxy)ethylsulfonic acid sodium salt.

Other suitable anionic monomers for the graft hydrogel polymer include sulfonic acids such as vinylsulfonic acid and 4-styrenesulfonic acid; (meth)acrylamidophosphonic acids such as (meth)acrylamidoalkylphosphonic acids (e.g., 2-acrylamidoethylphosphonic acid and 3-methacrylamidopropylphosphonic acid); acrylic acid and methacrylic acid; and carboxyalkyl(meth)acrylates such as 2-carboxyethylacrylate, 2-carboxyethylmethacrylate, 3-carboxypropylacrylate, and 3-carboxypropylmethacrylate. Still other suitable acidic monomers include (meth)acryloylamino acids, such as those described in U.S. 4,157,418 (Heilmann). Exemplary (meth)acryloylamino acids include, but are not limited to, N-acryloylglycine, N-acryloylaspartic acid, N-acryloyl-β-alanine, 2-acrylamidoglycolic acid, 3-acrylamido-3-methylbutyric acid. Salts of any of these acidic monomers can also be used.

In some embodiments, the nonwoven substrate has an ionic hydrogel polymer comprising cationic monomer units grafted to the surface of the nonwoven substrate. The polymer is grafted to the surface(s) of the nonwoven substrate by ionization initiated polymerization of "ionic grafting monomers" that are cationic (i.e., positively charged). Examples of useful nonwoven substrates comprising cationic monomer units grafted to the surface of the nonwoven substrate, and methods of grafting, are described, for example, in U.S. Published Pat. App. No. 2010/0155323 (Weiss et al., published June 24, 2010).

In some embodiments, the cationic monomer units are aminoalkyl(meth)acryloyl monomer units grafted to the surface of the nonwoven substrate. The polymer is grafted to the surface(s) of the nonwoven substrate by e-beam initiated polymerization of grafting monomers, which include aminoalkyl (meth)acryloyl monomers.

The grafting aminoalkyl (meth)acryloyl monomers are amino (meth)acrylates or amino (meth)acrylamides of Formula (III) or quaternary ammonium salts thereof.

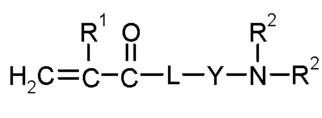

(III)

In Formula (III), $R^1$ is hydrogen or methyl, preferably methyl; L is -O- or - NH-; and Y is an alkylene (e.g., an

alkylene having 1 to 10 carbon atoms, 1 to 6, or 1 to 4 carbon atoms). Each $R^2$ is independently hydrogen or alkyl, preferably $C_1$-$C_4$ alkyl. Alternatively, the two $R^2$ groups taken together with the nitrogen atom to which they are attached can form a heterocyclic group that is aromatic, partially unsaturated (i.e., unsaturated but not aromatic), or saturated, wherein the heterocyclic group can optionally be fused to a second ring that is aromatic (e.g., benzene), partially unsaturated (e.g., cyclohexene), or saturated (e.g., cyclohexane). The counter ions of the quaternary ammonium salts may include, for example, halides, sulfates, phosphates, and nitrates. Such grafting monomers may be quaternary ammonium monomers (i.e., having a - $N(R^2)_3{}^+X^-$ group), wherein each $R^2$ is as defined, an $X^-$ is the counter anion. Such monomers having a quaternary ammonium group may be directly grafted to the surface of the nonwoven substrate or a grafting aminoalkyl (meth)acryloyl monomer, having a primary, secondary or tertiary amine group, may be grafted and subsequently converted to a quaternary ammonium group by alkylation.

In some embodiments of Formula (III), both $R^2$ groups are hydrogen. In other embodiments, one $R^2$ group is hydrogen and the other is an alkyl having 1 to 10, 1 to 6, or 1 to 4 carbon atoms. In yet other embodiments, the $R^2$ groups combine with the nitrogen atom to which they are attached to form a heterocyclic group. The heterocyclic group includes at least one nitrogen atom and can contain other heteroatoms such as oxygen or sulfur. Exemplary heterocyclic groups include, but are not limited to imidazolyl, piperazinyl, and morpholinyl. The heterocyclic group can be fused to an additional ring such as a benzene, cyclohexene, or cyclohexane. Exemplary heterocyclic groups fused to an additional ring include, but are not limited to, benzoimidazolyl.

Exemplary aminoalkyl (meth)acrylates (i.e., L in Formula (III) is oxy) include N,N-dialkylaminoalkyl(meth)acrylates such as, for example, N,N-dimethylaminoethylmethacrylate, N,N-dimethylaminoethylacrylate, N,N-diethylaminoethylmethacylate, N,N-diethylaminoethylacrylate, N,N-dimethylaminopropylmethacrylate, N,N-dimethylaminopropylacrylate, N-tert-butylaminopropylmethacrylate, N-tert-butylaminopropylacrylate and the like.

Exemplary amino (meth)acrylamides (i.e., L in Formula (III) is -NH-) include, for example, N-(3-aminopropyl)methacrylamide, N-(3-aminopropyl)acrylamide, N-[3-(dimethylamino)propyl]methacrylamide, N-methyl-N'-acryloylpiperazine, N-(3-imidazolylpropyl)methacrylamide, N-(3-imidazolylpropyl)acrylamide, N-(2-imidazolylethyl)methacrylamide, N-(1,1-dimethyl-3-imidazoylpropyl)methacrylamide, N-(1,1-dimethyl-3-imidazoylpropyl)acrylamide, N-(3-benzoimidazolylpropyl)acrylamide, and N-(3-benzoimidazolylpropyl)methacrylamide.

Exemplary quaternary salts of the aminoalkyl (meth)acryloyl monomers of Formula (III) include, but are not limited to, (meth)acrylamidoalkyltrimethylammonium salts (e.g., 3-[(methacryloylamino)propyl]trimethylammonium chloride (MAPTAC) and 3-[(acryloylamino)propyl]trimethylammonium chloride) and (meth)acryloxyalkyltrimethylammonium salts (e.g., 2-acryloxyethyltrimethylammonium chloride, 2-methacryloxyethyltrimethylammonium chloride, 3-methacryloxy-2-hydroxypropyltrimethylammonium chloride, 3-acryloxy-2-hydroxypropyltrimethylammonium chloride, and 2-acryloxyethyltrimethylammonium methyl sulfate).

In some embodiments, the nonwoven substrate has a hydrogel polymer comprising non-ionic monomer units grafted to the surface of the nonwoven substrate. The non-ionic grafted polymer contains ethylenically-unsaturated hydrophilic grafting monomer units; "non-ionic hydrophilic monomers". As used herein, "non-ionic hydrophilic monomers" are those polymerizable monomers having a water miscibility (water in monomer) of at least 1 wt.%, preferably at least 5 wt.% without reaching a cloud point. These monomers contain no groups that would retard the grafting polymerization. Examples of suitable non-ionic hydrophilic monomers include 2-hydroxyethyl(meth)acrylate (HEMA), 2-hydroxypropyl(meth)acrylate, 3-hydroxypropyl(meth)acrylate, 2, 3-dihydroxypropyl (meth)acrylate, 4-hydroxybutyl(meth)acrylate, N-vinyl caprolactam, N-vinyl acetamide, N-vinyl pyrrolidone, acrylonitrile, tetrahydrofurfuryl acrylate, acrylamide, mono- or di-N-alkyl substituted acrylamide, glycerol methacrylate, and combinations thereof. Preferred non-ionic hydrophilic monomers include 2-hydroxyethyl(meth)acrylate (HEMA), N-vinyl pyrrolidone, N-vinyl acetamide, methylacrylamide, dimethylacrylamide (DMA), and mixtures thereof.

In some embodiments, the non-ionic hydrophilic monomers are monofunctional ethylenically-unsaturated grafting monomer units having a poly(alkylene oxide) group. The monomer units having a poly(alkylene oxide) group is of the Formula (IV):

$$Z\text{-}Q\text{-}(CH(R^1)\text{-}CH_2\text{-}Q)_m\text{-}R^2 \qquad (IV)$$

wherein Z is a polymerizable ethylenically unsaturated moiety, $R^1$ is a H or $CH_3$, $R^2$ is a H, a $C_1$ to $C_4$ alkyl group, aryl group, or combinations thereof and m is from 2 to 100, preferably 5 to 20, and Q is a divalent linking group selected from -O-, $-NR^1-$ , $-CO_2$-and $-CONR^1$.

In one embodiment, the poly(alkylene oxide) group is a poly(ethylene oxide) (co)polymer. In another embodiment, the pendent poly(alkylene oxide) group is a poly(ethylene oxide-co-propylene oxide) copolymer. Such copolymers may be block copolymers, random copolymers, or gradient copolymers.

Useful ethylenically unsaturated moiety, Z, of the monomer may include:

| | |
|---|---|
| 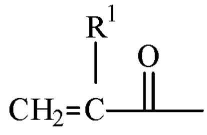 | 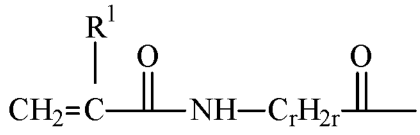 |
| 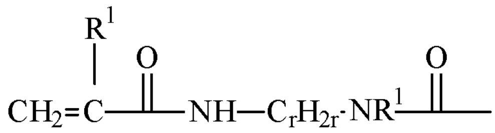 | $-CH=CH_2$, and $-C_rH_{2r}-CH=CH_2$ |

wherein $R^1$ is H or Me and r = 1-10.

The monomer having a poly(alkylene oxide) group can be prepared, for example, by reacting mono- or di-functional alkylene oxide (co)polymers (which are typically commercially available) with reactive ethylenically unsaturated compounds (e.g., acrylates). The functional groups terminating the poly(alkylene oxide) may include hydroxy groups, amine groups and carboxyl groups. A variety of reactive ethylenically unsaturated compounds such as acrylate derivatives can be used including, but not limited to, (meth)acrylic acid, (meth)acryloyl chloride, (meth)acrylic anhydride, and 2-isocyanatoethyl (meth)acrylate. Preferably, the monomer is prepared by reacting the mono- or di-functional alkylene oxide (co)polymer with (meth)acrylic anhydride. Typically, if a stoichiometric amount of the ethylenically unsaturated reactant is combined with the monofunctional alkylene oxide (co)polymer (such as a monohydroxy terminated alkylene oxide (co)polymer), 100% conversion to the monosubstituted product is obtained.

Examples of suitable monofunctional poly(alkylene oxide) monomers include poly(ethylene oxide) (meth)acrylate, polypropylene oxide) (meth)acrylate, poly(ethylene oxide-propylene oxide) (meth)acrylate, and combinations thereof. Such monomers preferably include one nonreactive end group such as ($C_1$-$C_4$) alkoxy, aryloxy (e.g., phenoxy), and ($C_1$-$C_4$) alkaryloxy. These groups can be linear or branched. These monomers can be of a wide range of molecular weights and are commercially available from sources such as Sartomer Company (Exton, PA); Shinnakamura Chemical Co., Ltd. (Tokyo, Japan); Aldrich (Milwaukee, WI); and Osaka Organic Chemical Ind., Ltd. (Osaka, Japan).

Typically, the grafted hydrogel polymer is uncrosslinked, and the imbibing solution containing the monomer mixture contains no polyethylenically unsaturated monomers (i.e., no crosslinkers).

In some embodiments, the grafted hydrogel polymer comprises ionic grafting monomers and/or non-ionic hydrophilic grafting monomers in any suitable combination of weight percentages totaling 100 wt.%, including the extreme values of 100 wt.% ionic grafting monomer or 100 wt.% non-ionic hydrophilic grafting monomer, as well combinations of ionic grafting monomers (cationic and/or anionic) and non-ionic hydrophilic grafting monomers any suitable amounts between these extremes, including, for example, having ionic grafting monomer in an amount of at least 1 wt.%, or at least 2 wt.%, or at least 5 wt.%, or at least 10 wt.%, or at least 30 wt.%, or at least 50 wt.%, or at least 70 wt.%, or at least 80 wt.%, or at least 90 wt.%, or at least 95 wt.%, or at least 98 wt.%, or even at least 99 wt.%, the remainder being non-ionic hydrophilic grafting monomer, each weight percentage relative to the weight of total monomer content.

In some embodiments, the total grafted monomer content may be from 0.5 to 5 times the weight of the nonwoven substrate. It is desirable to fill the interstitial spaces of the nonwoven substrate but not have the polymer chains bridge to link separate fibers of the nonwoven with grafted polymer strands, as this will restrict expansion of the nonwoven substrate and impede flux. One way to reduce this fiber-fiber bridging by the grafted polymer is to lower the monomer concentration for a given fiber size. It has been determined that the amount and the morphology of the grafted hydrogel polymer may be influenced by adding a water miscible organic solvent to the grafting imbibing solution to control the molecular weight of the grafted hydrogel polymer tendrils and reduce bridging of the tendrils.

The grafting of a hydrogel polymer onto the nonwoven substrate enhances water-absorption capacity of water-absorbent layer 180 of the present disclosure, which can be measured in terms of multiples of a weight of the grafted nonwoven substrate. The water-absorbent layer 180 comprising a nonwoven substrate having a grafted hydrogel polymer of the present description can absorb a weight of water that is at least 0.5 times, for example, at least 1 time, at least 2 times, at least 3 times, at least 4 times, at least 5 times, at least 10 times, at least 15 times, at least 20 times, or even at least 30 times the weight of water-absorbent layer 180 prior to absorbing water. The enhanced water-absorption capacity is useful for retaining an aliquot of water from an aqueous sample that is passed through water-absorbent layer 180, because a portion of the aliquot of water can be in fluid communication with microorganisms, water-soluble gelling agent, nutrients, and detection agents when present in filter plate article 100, in order to support growth and detection of microorganisms (if present). In some commercially available thin film culture devices, including those available from 3M Company (St. Paul, MN) under the trade designation "PETRIFIILM", a typical protocol calls for the addition of about 1 milliliter of water to the thin film culture device in order to support the growth and detection of microorganisms, whereas

in the filter plate article of the present description, filtration of an aqueous sample through water-absorbent layer 180 provides all of the water needed for the support the growth and detection of microorganisms. In some embodiments of filter plate article 100, it may be desirable for water-absorbent layer 180 to retain an aliquot of water from an aqueous sample wherein the volume of the aliquot of water is in a range from 0.1 milliliters to 2 milliliters, or from 0.3 to 1.5 milliliters, or from 0.5 to 1.5 milliliters, or from 0.7 to 1.3 milliliters, or from 0.8 to 1.2 milliliters, or even from 0.9 to 1.1 milliliters.

With regard to the grafting monomers *supra*, the monomers that are grafted to the surface of the nonwoven substrates usually have either an acrylate or other non-acrylate polymerizable functional group for grafting by e-beam irradiation. Acrylamide or methacrylate groups are preferred for grafting of the monomer to the nonwoven substrate surface (using the process described herein) due to the slower, more uniform reactivity and durability of such methacrylates or acrylamido moieties to nonwovens that have been exposed to e-beam irradiation.

As described in further detail below, functionalized substrates of the present disclosure may be prepared using above-described monomers to provide a grafted polymer on the surface of a porous nonwoven base substrate. When two or more of the above-described grafting monomers are used, the monomers may be grafted onto the nonwoven base substrate in a single reaction step (i.e., exposure to an ionizing radiation) followed by imbibing with all grafting monomers present or in sequential reaction steps (i.e., a first exposure to ionizing radiation followed by imbibing with one or more grafting monomer, then a second exposure to an ionizing radiation and a second imbibing after the second exposure to the ionizing radiation).

It will be further understood that the grafting process will yield a radical species on the surface of the nonwoven substrate. In some typical embodiments, after imbibing the nonwoven substrate with a solution containing a mixture of ionic and non-ionic grafting monomers, polymerization will initiate with the formation of a radical on the carbon alpha to the carbonyl of any one of the grafting monomers. The radical so formed may further polymerize with one or more additional grafting monomers, resulting in a grafted polymers having these groups pendent from the polymer chain as simply illustrated below.

$$\text{Substrate-}(M^{\text{ionic}})_x\text{-}(M^{\text{non-ionic}})_y$$

In the formula, the $-(M^{\text{ionic}})_x-$ represents the residue of the ionic grafting monomers of one of Formula (I), Formula (II), or Formula (III) having "x" polymerized ionic grafting monomer units, and the $-(M^{\text{non-ionic}})_y$ represents the polymerized non-ionic hydrophilic monomers, having y polymerized monomer units. The polymer may be random or block. The polymer may be directly grafted via the residue of the $-(M^{\text{ionic}})-$ monomer, as shown, or may be directly grafted via the $-(M^{\text{non-ionic}})-$ non-ionic hydrophilic monomer.

The values of the subscripts x and y may be an integral or non-integral value and correspond to the amount of each monomer in the imbibing solution previous described. For example, in some embodiments the value of the subscript "x" will correspond to an amount of from 70 wt.% to 100 wt.% of the ionic grafting monomers in the imbibing solution, and the value of subscript y will correspond to an amount of from 30 wt.% to 10 wt.% of the non-ionic hydrophilic monomers.

In some other embodiments, the nonwoven substrate may be grafted exclusively with non-ionic monomers selected from the "non-ionic hydrophilic monomers" described above, to provide a nonwoven substrate having a non-ionic grafted hydrogel polymer, and the value of the subscript y will correspond to 100 wt.% of the non-ionic hydrophilic monomers, and the values of the x subscript will correspond to 0 wt.%.

It will be understood that various combinations of values for the subscripts x and y may be used in order to provide a nonwoven substrate having a grafted hydrogel that is capable of maintaining a water activity ($A_W$)value of greater than 0.91 for article 100 in the methods described herein for using article 100.

The process of preparing the grafted nonwoven substrate comprises providing a nonwoven substrate, exposing the nonwoven substrate to e-beam radiation in an inert atmosphere, and subsequently imbibing the exposed substrate with a solution comprising grafting monomers to graft polymerize said grafting monomers to the surface of the nonwoven substrate. Suitable methods for preparing the grafted nonwoven substrate include those described, for example, in U.S. Published Pat. App. Nos. 2007/0154651 (Weiss et al.) and 2010/0075560 (Seshadri et al.).

In some embodiments of radiation grafting, the first step includes exposing the nonwoven substrate to ionizing radiation, such as e-beam radiation, in an inert atmosphere. Generally, the substrate is placed in a chamber purged of oxygen. Typically, the chamber comprises an inert atmosphere such as nitrogen, carbon dioxide, helium, argon, etc., with a minimal amount of oxygen (less than 100 ppm), which is known to inhibit free-radical polymerization.

The irradiation step comprises the ionizing irradiation of nonwoven substrate surfaces, preferably with ionizing e-beam or gamma radiation to prepare free radical reaction sites on such surfaces upon which the monomers are subsequently grafted. "Ionizing irradiation" means radiation of a sufficient dosage and energy to cause the formation of free radical reaction sites on the surface(s) of the base substrate. Ionizing radiation may include gamma, electron-beam, x-ray and other forms of electromagnetic radiation. In some instances, corona radiation can be sufficiently high energy radiation. The radiation is sufficiently high energy, that when absorbed by the surfaces of the base substrate, sufficient

energy is transferred to that support to result in the cleavage of chemical bonds in that support and the resultant formation of free radical sites on the nonwoven substrate. One or more layers of nonwoven substrates may be subjected to the ionizing radiation.

High energy radiation dosages are measured in units of kilogray (kGy). Doses can be administered in a single dose of the desired level or in multiple doses which accumulate to the desired level. Dosages can range cumulatively from 1 kGy to 200 kGy. The dose can be delivered all at once such as from an E-beam source or accumulated from a slow dose rate over several hours such as dosage delivered from a gamma source. Preferably, the cumulative dosage exceeds 20 kGy (2 Mrads) for substrates resistant to radiation damage.

Electron beam is one preferred method of grafting due to the ready-availability of commercial sources. Electron beam generators are commercially available from a variety of sources, including the electron beam generator available from Energy Sciences, Inc. (Wilmington, MA) under the trade designation "ESI ELECTROCURE EB SYSTEM", and including the electron beam generator available from PCT Engineered Systems, LLC (Davenport, IA) under the trade designation "BROADBEAM EB PROCESSOR". For any given piece of equipment and irradiation sample location, the dosage delivered can be measured in accordance with ASTM E-1275 entitled "Practice for Use of a Radiochromic Film Dosimetry System."

Dose is the total amount of energy absorbed per mass unit. Generally, it was found that doses in the range of 20 to 40 kGy were suitable for generating the grafted hydrogel polymer.

Other sources of irradiation, such as gamma radiation sources, may be used with equal grafting performance. Generally, suitable gamma ray sources emit gamma rays having energies of 400 keV or greater. Typically, suitable gamma ray sources emit gamma rays having energies in the range of 500 keV to 5 MeV. Examples of suitable gamma ray sources include cobalt-60 isotope (which emits photons with energies of approximately 1.17 and 1.33 MeV in nearly equal proportions) and cesium-137 isotope (which emits photons with energies of approximately 0.662 MeV). The distance from the source can be fixed or made variable by changing the position of the target or the source. The flux of gamma rays emitted from the source generally decays with the square of the distance from the source and duration of time as governed by the half-life of the isotope.

Without intending to be limited to any particular mechanism, it is believed that the exposure of the nonwoven substrate to ionizing radiation results in free radical sites on the substrate surface which can then subsequently react with the grafting monomers in the imbibing step.

The total dose received by the nonwoven substrate primarily affects the number of radical sites formed on the surface thereof and subsequently the extent to which the grafting monomers are grafted onto the nonwoven substrate.

In some embodiments of the instant method, the irradiated substrate, having free radical sites on the surface of the nonwoven substrate, is imbibed with the monomer solution subsequent to and not concurrent with, the irradiation step. The free radical sites generated on the surface of the nonwoven substrate have average lifetimes ranging from several minutes to several hours and progressively decay to a low concentration within about ten hours at room temperature. Lower temperatures, such as dry ice temperatures, promote longer radical lifetimes. Alternatively, humidification and nitrous oxide can increase the rate of substrate radical formation via generation of hydroxyl radicals.

In some embodiments, the irradiated nonwoven substrate is imbibed with the monomer solution immediately after the irradiation step. Generally when using E-beam the irradiated substrate is imbibed within an hour, preferably within ten minutes. Generally, when using gamma as a source, the substrate should be imbibed immediately after irradiation since irradiation residence time will be long.

In the imbibing step, the nonwoven substrate is contacted with the imbibing solution containing one or more grafting monomers and in amounts previously described. Suitable methods of imbibing include, but are not limited to, spray coating, flood coating, knife coating, Mayer bar coating, dip coating, and gravure coating.

The imbibing solution remains in contact with the nonwoven substrate for a time sufficient for the radical sites to initiate polymerization with the grafting monomers. When imbibed with a solution of monomers, grafting reactions are mostly completed after 12 hours exposure; generally resulting in about 50+ percent conversion of monomers to grafted polymer. As a result, the nonwoven substrate comprises grafted polymers and/or copolymers attached to the interstitial and outer surfaces of the nonwoven substrate.

In some other embodiments of radiation grafting the nonwoven substrate, an embodiment of a "direct radiation grafting" method comprises: 1) providing a nonwoven substrate of the present disclosure; 2) imbibing the nonwoven substrate with a solution comprising one or more grafting monomers having at least one acryloyl group; and 3) exposing the imbibed nonwoven substrate to ionizing radiation, preferably e-beam or gamma radiation, so as to form a functionalized substrate comprising a nonwoven substrate having grafted monomer.

In embodiments of grafting the nonwoven substrate, the grafting monomers have a free-radically polymerizable group. The free-radically polymerizable group is typically an ethylenically unsaturated group such as a (meth)acryloyl group or a vinyl group. The free-radically polymerizable group typically can react with the surface of the nonwoven substrate when exposed to an electron beam or other ionizing radiation. That is, reaction of the free-radically polymerizable groups of the grafting monomers with the surface of the nonwoven substrate in the presence of the electron beam results

in the formation of grafted species attached to the nonwoven substrate. One or more grafting monomers may be grafted onto interstitial and outer surfaces of the nonwoven substrate.

In an embodiment of a method that combines "direct radiation grafting" and "UV grafting" techniques comprises: 1) providing a nonwoven substrate of the present disclosure; 2) imbibing the nonwoven substrate with a first solution to form an imbibed nonwoven substrate, the first solution comprising (a) at least one grafting monomer having an acrylate group and a photoinitiator group and (b) one or more monomers having at least one (meth)acryloyl group and at least one additional ethylenically unsaturated, free-radically polymerizable group; and optionally (c) one or more additional monomers having at least one free-radically polymerizable group; 3) exposing the imbibed nonwoven substrate to a controlled amount of electron beam radiation so as to form a first functionalized substrate comprising grafted photoinitiator groups attached to the surfaces of the nonwoven substrate; 4) optionally, imbibing the substrate comprising grafted photoinitiator groups with a second solution comprising one or more additional grafting monomers; and 5) exposing the nonwoven substrate comprising grafted photoinitiator groups to a controlled amount of UV radiation to polymerize or crosslink the remaining ethylenically unsaturated, free-radically polymerizable groups.

As discussed above, the imbibing solution may comprise one or more grafting monomers suitable for grafting onto surfaces of the nonwoven substrate. Any of the exemplary grafting monomers described above can be included in the imbibing solution. In addition to the described grafting monomers, the imbibing solution can contain other materials such as, for example, one or more surfactants, dyes, pigments and solvents.

The concentration of each grafting monomer in the imbibing solution may vary depending on a number of factors including, but not limited to, the grafting monomer or monomers in the imbibing solution, the extent of grafting desired, the reactivity of the grafting monomer(s), and the solvent (if present). Typically, the total concentration of the monomers in the imbibing solution ranges from about 1 wt.% to about 100 wt.%, desirably, from about 5 wt.% to about 30 wt.%, and more desirably from about 15 wt.% to about 25 wt.% based on a total weight of the imbibing solution.

The imbibing solution further comprises an aqueous blend of a water miscible organic solvent and the grafting monomer(s). It has been found that the solvent blend influences the morphology of the grafted polymer and the resulting flux rate when used in separation applications. The ratio of water to organic solvent can vary widely, but is typically greater than 1: 1 (v/v) water to organic solvent, preferably greater than 5:1 (v/v), and more preferably greater than 7:1 (v/v). The ratios are generally adjusted so that the resulting grafted nonwoven substrate produces pressure and flux responses suitable for the filtration of an aqueous sample through filter assembly 150.

In some embodiments, the water miscible solvents are protic group containing organic liquids such as the lower alcohols having 1 to 4 carbon atoms, lower glycols having 2 to 6 carbon atoms, and most preferably lower glycol ethers having 3 to 6 carbon atoms and 1 to 2 ether linkages. In some embodiments higher glycols such as poly(ethylene glycol) may be used. Specific examples are methanol, ethanol, n-butanol, t-butyl alcohol, ethylene glycol, methoxyethanol, ethoxyethanol, propoxyethanol, butoxyethanol, methyl carbitol, ethyl carbitol, and mixtures thereof.

In other embodiments, non-protic water miscible organic solvents that can also be used include ketones, amides, and sulfoxides, including, for example, acetone, methyl ethyl ketone, dimethylformamide, dimethylacetamide, N-methylpyrrolidone, and dimethyl sulfoxide.

Once the nonwoven substrate has been imbibed for a desired period of time, the nonwoven substrate bearing grafted polymer groups may be optionally rinsed and/or dried.

In the optional rinsing step, the functionalized nonwoven substrate is washed or rinsed one or more times to remove unreacted monomers, nongrafted oligomers or polymers, solvent or other reaction by-products. Typically, the functionalized substrate is washed or rinsed up to three times using a water rinse, an alcohol rinse, a combination of water and alcohol rinses, and/or a solvent rinse (e.g., acetone, methyl ethyl ketone, etc). When an alcohol rinse is used, the rinse may include one or more alcohols including, but not limited to, isopropanol, methanol, ethanol, or any other alcohol that is practical to use and an effective solvent for any residual monomer. In each rinse step, the functionalized substrate may pass through a rinse bath or a rinse spray. In some embodiments, the rinse may comprise an ionic buffer solution that would reduce swelling of the hydrogel, the amount of retained water, and also avoiding weakening the nonwoven substrate during this rinse step.

In the optional drying step, the functionalized substrate is dried to remove any rinse solution. Typically, the functionalized substrate is dried in oven having a low oven temperature for a desired period of time (referred to herein as "oven dwell time"). Oven temperatures that are "low oven temperatures" typically range from about 60°C to about 120°C, while oven dwell times typically range from about 2 minutes to about 5 minutes. Any conventional oven may be used in the optional drying step. It should also be noted that in other embodiments the drying step can proceed before the rinsing step to eliminate volatile components before extraction of non-grafted residue. Following the optional drying step, the dried functionalized substrate can be taken up in roll form to be stored for future use.

In yet another method suitable for grafting a hydrogel polymer onto a nonwoven substrate, a UV grafting process may be used wherein the nonwoven substrate is first imbibed with grafting monomers of Formula I, Formula II, or Formula III, including any non-ionic hydrophilic monomers of the present disclosure, followed by UV irradiation, to produce a nonwoven substrate having grafted hydrogel polymer.

In an embodiment of a method of using the filter plate article of the present description, the method includes: providing the filter plate article of the present description; providing an aqueous sample; passing the aqueous sample firstly through the microporous membrane and secondly through the water-absorbent layer, wherein the water-absorbent layer retains an aliquot of water from the aqueous sample; incubating the filter plate article for an incubation period, wherein at least a portion of the aliquot of water contacts the microporous membrane throughout the incubation period; and observing an indication of the presence or absence of microbial growth.

In some embodiments, the method further comprises: providing a filtration apparatus, the filtration apparatus comprising a filter seat comprising a filter support surface and defining a filter seat inlet and a filter seat outlet; and placing the water-absorbent layer of the filter plate article against the filter support surface prior to passing the aqueous sample firstly through the microporous membrane and secondly through the water-absorbent layer. In some further embodiments, placing the water-absorbent layer of the filter plate article against the filter support surface comprises: inserting the filter assembly into the filtration apparatus along an insertion path defined by first and second guide members arranged parallel to each other on opposing sides of the filter seat and lateral to the filter support surface.

In some embodiments of the method, the incubation period is at least 1 hour, at least 2 hours, at least 4 hours, at least 8 hours, at least at least 12 hours, at least 16 hours, at least 20 hours, or even at least 24 hours.

In some embodiments of the method, the aliquot of water has a volume in a range from 0.5 milliliter to 1.5 milliliter, from 0.8 milliliter to 1.3 milliliter, or even from 0.9 milliliter to 1.1 milliliter.

In a typical filtering operation of the present disclosure, filter assembly 150 of article 100 is placed on or into a filtration apparatus to aid with passing an aqueous sample firstly through the microporous membrane and secondly through the water-absorbent layer. Typically, cover sheet 140 and base member 110 are both peeled back from filter assembly 150 prior to passing an aqueous sample firstly through the microporous membrane and secondly through the water-absorbent layer.

Article 100 may be used in conjunction with any suitable filtration apparatus, including, for example, a manual clamp-type filtration apparatus that can hold a filter membrane between a sample reservoir and a filtrate collection reservoir, as is commonly used in the filtration of mobile phase liquids for high performance liquid chromatography. A suitable filtration apparatus includes, for example, that clamp-type filtration apparatus available from Sigma-Aldrich (St. Louis, MO) under the trade designation "SUPELCO MOBILE PHASE FILTRATION APPARATUS"). It will be understood that a suitable positioning of a clamp on this type of filtration apparatus can hold the composite filter body 155 between the sample reservoir and the filtrate collection reservoir, and that typically this clamp-type filtration apparatus is connected to a vacuum source to aid in passing an aqueous sample from the sample reservoir, through composite filter body 155, and into the filtrate collection reservoir, while retaining an aliquot of water from the aqueous sample in water-absorbent layer 180.

FIGS. 5, 6A, 6B, 7A, and 7B collectively show an exemplary embodiment of filtration apparatus 10 of the present description that may be used with article 100 of the present description. This type of filtration apparatus is described in U.S. Provisional Pat. App. No. 61/360489 (Miller et al.), filed June 30, 2010, which describes various embodiments of a filtration apparatus useful in the present method. In the exemplary embodiment shown in FIG. 5, filtration apparatus 10 is mounted upright on posts 2 and 3, which in turn are mounted on base 1; however, filtration apparatus 10 need not be mounted in this fashion, and may be mounted in any suitable manner including, for example, being mounted in a housing that includes front and back walls that have any necessary openings to admit a filter plate device, a sample to be filtered, or a vacuum connection. Filtration apparatus 10 includes a filtration assembly and a guide assembly. The filtration assembly includes sample head 15 and filter seat 12 slidably engageable with sample head 15. Sample head 15 is shown as being mounted on top support plate 7, having sample head top portion 15' extending above top support plate 7, and defining sample head inlet 16.

Filter seat 12 rides on shaft 35 that slides through an opening in bottom support plate 6, permitting filter seat 12 to engage sample head 15. Shaft 35 may be engaged with any suitable handle or mechanism to move filter seat 12 towards or away from sample head 15. Filter seat 12 includes a filter support surface 11, and sample head 15 has a sample head outlet (see 17 in FIG. 6A) that the faces filter support surface 11. In an open position, filter seat 12 and sample head 15 define an insertion gap therebetween.

The guide assembly in the exemplary embodiment shown in FIG. 5 of filtration apparatus 10 includes first guide member 50 and second guide member 52. First guide member 50 and second guide member 52 are arranged parallel to each other on opposing sides of the filtration assembly to define an insertion path, thereby to guide filter assembly 150 into the insertion gap. First guide member 50 and second guide member 52 are notches defined in side walls 4 and 5. Alternatively, other embodiments of first and second guide members may be used, including, for example, slots defined in walls 4 and 5, guide rails mounted on portions of a housing, combinations of these guide members, or any other guide members suitable for guiding a filter plate device into the insertion gap. Typically, first guide member 50 comprises a first end and a second end, and second guide member 52 comprises a first end and a second end, and the first ends of the first and second guide members are oriented toward the front of filtration apparatus 10 to define an insertion slot arranged to allow a user to insert filter assembly 150 of article 100 along the insertion slot into filtration apparatus 10.

Filter seat 12 is shown to include groove 70 suitable for mounting an O-ring (not shown) to aid in sealing filter seat 12 against a filter membrane layer inserted into the insertion gap. Filter seat 12 also includes takeoff adapter 60 for connection to a filtrate collection apparatus (not shown), which may optionally include a vacuum source (not shown).

FIGS. 6A and 6B are each a front view of filtration apparatus 10, excluding any additional mounting components. In FIG. 6A, filter seat 12 has filter support surface 11, which may include a sintered glass frit, or a metal screen, or other suitable filter support component that permits a liquid sample, generally an aqueous sample, to flow through with less resistance than a filter plate device to be used with the filtration apparatus. Filter seat 12 defines a filter seat channel 14 extending from a filter seat inlet 20 to a filter seat outlet 30. Filter seat outlet 30 leads to takeoff adapter 60 (shown in FIG. 5). Filter seat 12 is mounted on a shaft 35 having a toggle mechanism represented by toggle handle 40. As shown in FIG. 6B, filter seat 12 is slidably engageable with sample head 15, where in the illustrated embodiment toggle handle 40 is toggled from an gap open position to a gap closed position, corresponding to an open insertion gap position (FIG. 6A) and a closed gap position (FIG. 6B), respectively. It will be understood that when the filtration apparatus is in an open gap position, a filter membrane layer of a filter card may be inserted into the insertion gap.

A suitable exemplary embodiment of toggle mechanism 40 may include a toggle clamp, a nonlimiting example of which is a Vertical Hold-Down Toggle Locking Clamp available from DESTACO Workholding (Auburn Hills, MI). However, other mechanisms for moving shafts similar to shaft 35 are well known in the art, including for example hydraulic or pneumatic actuators, which may include a convenient option of being activated by a footswitch. In the remaining figures, the mechanism for moving shaft 35 is omitted, but it will be understood that such a mechanism is readily supplied.

FIG. 7A depicts an embodiment of article 100 and filtration apparatus 10 being brought together along a path indicated by A and A' that is aligned with first and second guide members 50 and 52, respectively. Article 100 is shown as having cover sheet peripheral boundary 149 and base member peripheral boundary 119 each peeled back from filter assembly 150, which includes peripheral boundary 159, here shown as aligned with the path indicated by A and A'. FIG. 7A also shows first and second cover sheet support portions 80 and 82, as well as first and second base member support portions 81 and 83. FIG. 7B shows article 100 having filter assembly 150 inserted into filtration apparatus 10, wherein cover sheet peripheral boundary 149 abuts first and second cover sheet support portions 80 and 82, and wherein base member peripheral boundary 119 abuts first and second base member support portions 81 and 83. First and second cover sheet support portions 80 and 82, and first and second base member support portions 81 and 83 may each comprise a ramp portion (not shown) to aid in abutting cover sheet peripheral boundary 149 and base member peripheral boundary 119 against filtration apparatus 10.

In the exemplary embodiments shown in FIGS. 5, 6A, and 7A, filter seat 12 and sample head 15 are in an open position, and when filter assembly 150 is inserted into filtration apparatus 10, composite filter body 155 becomes aligned in fluid communication with filter support surface 11. Typically, filter seat 12 is then urged towards sample head 15 into a closed position, as in FIG. 6B, and contacts water-absorbent layer 180. An aqueous sample is provided, and the aqueous sample is introduce through sample head 15, then firstly passing through microporous membrane 160 and secondly through water-absorbent layer 180. Optionally, a vacuum source is attached to takeoff adapter 60 to facilitate flow of the aqueous sample through composite filter body 155, and into filter seat 12. A typical pressure value for the optional vacuum source is in a range of 30 kiloPascals (kPa) to 50 kPa. Optionally, the aqueous sample may be injected via syringe through a suitable adapter on sample head 15, with sufficient pressure to facilitate flow of the aqueous sample through composite filter body 155. When the aqueous sample is no longer passing through microporous membrane 160, filter seat 12 is typically urged away from sample head 15 and article 100 is withdrawn from filtration apparatus 10. As mentioned above, water-absorbent layer 180 retains an aliquot of water from the aqueous sample. Typically, cover sheet 140 and base member 110 are each then brought into contact with filter assembly 150. A spreader device may be used to press cover sheet 140 and base member 110 against filter assembly 150 to assure intimate contact of water from the aliquot of water with any optionally included first dry coating 116 (and/or second dry coating 146), thereby wetting the components of those dry coatings, if present.

In embodiments where sample head 15 is fixed (i.e., not a disposable component), it will typically be desirable to rinse the sample head channel and sample head outlet prior to carrying out another filtration.

FIG. 8 shows an embodiment of the present disclosure wherein a plurality of filtration apparatuses 10 and 10' are arranged to share a wall 504, and the wall 504 defining a first guide member 510 having cover support surfaces 582 and 583 above and below a front end of first guide member 510, with a similar structure repeated on either side. Not shown, but will be understood, is a set of actuators connected to shafts 535 and 535', making possible a parallel operation of the plurality of filtration devices.

FIG. 9 shows an alternate embodiment of a filtration apparatus, wherein cover support portions 80, 81, 82, and 83 each include an optional ramp portion, shown as ramp portions 780, 781, 782 and 783, respectively. In some embodiments, all four ramp portions may be present, while in other embodiments it may be desirable to include only the lower ramp portions 781 and 783, or only the upper ramp portions 780 and 782. Selection of which optional configuration of ramp portions to include may depend in part on factors related to the flexibility or stiffness of cover layers 140 and 110.

FIG. 10 shows a side view of an embodiment of a filter seat 12 showing details also described in FIG. 6A,

including a side view of filter seat channel 14. Also shown is attachment portion 33, which may be of a different material than filter seat 12. In some embodiments, filter seat 12 may be made of a plastic, e.g., a polycarbonate or an epoxy material, while attachment portion 33 may be made of a metal, e.g., brass, and these two portions may be connected by any standard means, for example, by using screws. Groove 70 is provided to accommodate an O-ring (not shown), which may be an important factor in providing a tight seal against a filter membrane layer inserted into the insertion gap when the filter seat is slidably engaged into the closed insertion gap position. A suitable O-ring may have a treated surface, for example a silicone surface, and commercially available examples are readily available from, for example, Marco Rubber and Plastic Products, Inc. (North Andover, MA). The treated O-ring may provide a better seal, better chemical wear properties, and better release from surface than a non-treated rubber O-ring.

FIG. 11 shows an alternate embodiment of a filtration apparatus that makes use of a disposable cartridge 900. In the embodiment shown, it is clear that disposable cartridge 900 may be part of a disposable syringe having a plunger 902, and a tip portion 992 that fits into an adapter fitting 990, which may include, for example, a Luer lock adapter fitting. Other types of disposable cartridges and adapter fittings are available and may be useful. Useful types of fittings to engage a disposable cartridge may include ball detent fittings, Luer lock fittings, bayonet fittings, threaded fittings, and other types of fittings commonly known in the art.

Depending on the type of cartridge and fitting used, it may be that a tip portion 902 makes contact against the filter membrane layer. In such instances, it may be desirable to include a surface coating on tip portion 902, including, for example, a fluorochemical coating. The coating may provide benefit in terms of clean separation between the disposable cartridge and the filter membrane layer.

Also shown in FIG. 11 is an optional spring member 959, positioned about midway along the insertion path, and provided to aid in urging a filter membrane layer away from sample head outlet 17 (refer to FIG. 6A) after the sample has been filtered and the filter seat is moved to an open insertion gap position. Typically, a spring member 959 would be provided adjacent to each of the first and second guide members, in order to symmetrically displace the filter membrane layer down away from sample head outlet 17.

It will be understood that the filtration apparatus of the present disclosure may be useful in carrying out filtrations where instead of using a filter plate device, a filter membrane is used, of which many types are well known.

If the optional vacuum source is employed to facilitate flow of the aqueous sample through composite filter body 155, article 100 is typically separated from the optional vacuum source within a short period of time, typically less than 30 seconds, or less than 20 seconds, or less than 10 seconds, or even less than 5 seconds. Separation from the optional vacuum source may include, for example, removing article 100 from the filtration apparatus, closing a valve between takeoff adapter 60 and the vacuum source, or switching off the vacuum source. It will be understood that if the vacuum source is allowed to actively draw a vacuum against composite filter body 155 for a prolonged period (i.e., longer than 60 seconds, although the amount of time may depend upon the level of vacuum pressure) after the aqueous sample has passed through microporous membrane 160, the water activity ($A_W$) value of composite filter body 155 may diminish.

After the above filtration operation, article 100, including the aliquot of water from the aqueous sample and any microorganisms that may have been captured on microporous membrane 160, is incubated for an incubation period, in order to permit growth of microorganisms (if present).

It will be understood that the incorporation of water-absorbent layer 180 into filter plate article 100 greatly simplifies the operation of processing an aqueous sample for the detection of microorganisms therein, with no additional hydration of media needed. Additionally, there is a reduced potential for inadvertent contamination during hydration of the filter plate article, as compared with, for example, the need to hydrate other culture devices with water from an additional source.

The method further comprises incubating article 100 for an incubation period, wherein at least a portion of the aliquot of water contacts the microporous membrane throughout the incubation period. The incubation provides the conditions (i.e., time, temperature) to facilitate the growth of a microorganism, if present. A person of ordinary skill in the relevant art will recognize that the incubation temperature may be selected according to the microorganism to be detected. For example, if a yeast or mold is to be detected, the first incubation temperature typically may be from about 23°C to about 32°C. For example, if a bacterium is to be detected, the incubation temperature typically may be from about room temperature to about 45°C.

According to the present disclosure, the incubation period may be as short as one hour. In some embodiments, the first incubation is less than 2 hours, less than 3 hours, less than 4 hours, less than 5 hours, less than 6 hours, less than 7 hours, less than 8 hours, less than 9 hours, less than 10 hours, less than 11 hours, less than 12 hours, less than 13 hours, less than 14 hours, less than 15 hours, less than 16 hours, less than 24 hours, less than 36 hours, or even less than 48 hours.

Contact between the hydrated region of the microporous membrane and the second dry coating permits hydration of second dry coating 146 and, thereby, an interaction of a detection reagent, a lysis agent, or a selective agent in second dry coating 146 with a microorganism (if present), a component of the microorganism, and/or a metabolic byproduct of the microorganism. The interaction can provide a detectable signal.

The method further comprises observing an indication of the presence or absence of microbial growth. The indication may be observable visually and/or by an instrument (e.g., an imaging system) and both types (manual and automated) of detection are contemplated by the present disclosure.

The nature of the indication of microbial growth generally is dependent upon the detection reagent (or plurality of detection reagents) in article 100. For example, certain detection reagents (e.g., enzyme substrates) produce a detectable colored or fluorescent product when they interact with a microbe or a component thereof. For example, certain detection reagents (e.g., pH indicators) produce a detectable change in color or fluorescence in the presence of acidic or basic metabolic products of microorganisms. For example, certain detection reagents (e.g., polysaccharide or polypeptide polymers) are relatively opaque in their native state and become relatively transparent when degraded by microorganisms or components thereof.

In some embodiments, an indication of the presence of microbial growth comprises the presence of a detectable microbial colony. The microbial colony may be detected visually. The microbial colony may be detected using an imaging system. Suitable systems for detecting microbial colonies are described, for example, in International Patent Publication No. WO 2005/024047 (Graessle et al.); U.S. Patent Application Publication Nos. 2004/0101954 (Graessle et al.) and 2004/0102903 (Graessle et al.); and U.S. Patent Application Serial No. 61/187,107 (Attorney Docket No. 65250US002), filed June 15, 2009. Suitable imaging systems include, for example, that plate reader available from 3M Company (St. Paul, MN) under the trade designation "PETRIFILM PLATE READER", that colony counter available from Spiral Biotech (Norwood, MA) under the trade designation "PETRISCAN", and those plate scanners available from Synbiosis (Cambridge, U.K.) under the trade designations "PROTOCOL" and "ACOLYTE".

In some embodiments, an indication of the presence of microbial growth comprises the presence of a reaction, detected visually or by instrument, associated with a non-visually-detectable microbial colony.

In any one of the above embodiments, the method can further comprise enumerating microorganisms. In some embodiments, the microorganisms can be enumerated by counting the number of discrete colonies in the article. In some embodiments, the microorganisms can be enumerated by counting the number of discrete reactions (e.g., colored zones, fluorescent zones, clear zones) that are associated with non-visually-detectable colonies in the article. Enumerating microorganisms may comprise using an imaging system to enumerate microorganisms.

Observing an indication of the presence or absence of microbial growth may comprise obtaining an image of the article. Observing an indication of the presence or absence of microbial growth may further comprise printing, displaying, or analyzing the image.

The invention will be further illustrated by reference to the following non-limiting Examples. All parts and percentages are expressed as parts by weight unless otherwise indicated.

## Examples

Porosity Test Method

Mean flow pore size was measured according to ASTM F316-03 Standard Test Methods for Pore Size Characteristics of Membrane Filters by Bubble Point and Mean Flow Pore Test Method B using a test fluid available from DuPont (Wilmington, DE) under the trade designation "FREON TF".

Water-Absorption Capacity Test Method

A 50 milliliter sample of phosphate-buffered saline was drawn through a 47 millimeter diameter composite filter body including a microporous membrane and a water-absorbent layer (see, for example, Preparative Example C below), using vacuum filtration with vacuum pressure of 85 kilopascal during the flow of water through the composite filter body. Weights of the composite filter body before and after filtration were obtained, and a Water-Absorption Capacity Ratio was calculated, using a weight of water retained in the composite filter body compared to a weight of the water absorbent layer (calculated as a weight of the composite filter body minus a typical mass of components other than the water-absorbent layer):

$$C_{WA} = [(A - B)/(B - K)]$$

where:

$C_{WA}$ = Water Absorption Capacity Ratio
A = final mass of composite filter body and water
B = initial mass of composite filter body, and

K = typical mass of composite filter body components, excluding water-absorbent layer

In a variant of the Water-Absorption Capacity Test Method, called the "Water-Absorbent Layer Capacity Test Method", the weight of the water absorbent layer alone is measured prior to incorporation of the water-absorbent layer into the composite filter body, and the Water-Absorption Capacity ratio ($C_{WA}$) is calculated using the measured weight of the water-absorbent layer:

$$C_{WA} = [(A - B)/D]$$

where:

$C_{WA}$ = Water Absorption Capacity Ratio
A = final mass of composite filter body and water
B = initial mass of composite filter body, and
D = initial mass of water-absorbent layer, prior to incorporation in composite filter body

Preparative Example A (Nylon Nonwoven Substrate)

Using the method described in paragraph [0146] of U.S. Published Pat. App. No. 2010/0155323 (Weiss et al.), published June 24, 2010, a nylon nonwoven substrate was prepared wherein a nylon-6 available from BASF Corporation Engineering Plastics (Wyandotte, MI) under the trade designation "B-3" was used to produce meltblown nonwoven substrate. The melt temperature was 265 °C with a mass flow rate of 0.25 gram/hole/minute on a standard meltblowing drilled orifice die. Hot air at 1000 SCFM (28 CMM per meter of die width) at a temperature of 350°C was used to attenuate the fibers. The fibers were collected 0.43 meter away from the die on a foraminous stainless steel belt and were bonded under 200°C air drawn through the web at 137 (meters per minute) face velocity for 0.14 second followed by cooling air at 29°C at the same face velocity for 0.8 second. The collected web was 50 grams per square meter and had an effective fiber diameter of 5.8 micrometers. The collected web had a thickness of 0.81 mm before calendaring between two 25 centimeter diameter smooth steel rolls set at 82°C running at 1.5 meter/minute with a nip pressure of 176 Newtons per lineal centimeter (N/lcm) of web. The resulting web thickness was 0.25 millimeter.

Preparative Example B (Water-Absorbent Nylon Nonwoven Layer)

Using the method described in paragraphs [0153]-[0155] of U.S. Published Pat. App. No. 2010/0155323 (Weiss et al.), published June 24, 2010, a 30 centimeter by 43 centimeter sample of the nylon nonwoven substrate of Preparative Example A was purged of air under a nitrogen atmosphere in a glove box and inserted into a "ZIPLOC" plastic bag and sealed. The sealed bag was then removed from the glove box and irradiated to a dose level of 40 kGy by passing it through the electron beam set with an accelerating voltage of 300 kV and a web speed of 6 m/min (20 feet/minute). After returning the sealed bag to the nitrogen atmosphere-controlled glove box, the irradiated nonwoven substrate was removed and placed inside a non-irradiated, nitrogen purged, "ZIPLOC" bag.

The freshly prepared nonwoven sample was imbibed with 100 grams of the nitrogen purged imbibing solution comprising 15 wt.% 3-[(methacryloylamino)propyl]trimethylammonium chloride (MAPTAC) monomer, 15 wt.% methanol, 10 wt.% polyethylene glycol with hydroxy end groups, average molecular weight 4,000 g/mole (PEG 4000) and 60 wt.% water, and the bag resealed after expelling most of the nitrogen. During this step the oxygen level within the glove box was generally maintained below 40 parts per million (ppm).

The sample was maintained flat in the bag and evenly saturated for four hours by occasionally rotating the bag. The resulting grafted nylon nonwoven substrate was removed from the bag and carefully washed by soaking it for 10 minutes in a tray containing 2 liters of fresh deionized water. The substrate was removed from the tray, compressed between multiple layers of paper towels and the washing process repeated two more times with fresh DI water and air dried, to provide the water-absorbent nylon nonwoven layer.

Preparative Example C (Composite Filter Body and Demonstration of Water-Absorption)

A 47 millimeter polycarbonate microporous membrane obtained from Whatman Inc. (Florham Park, NJ) under the trade designation "WHATMAN POLYCARBONATE MEMBRANE FILTER NO. 111107" was laid on top of a similar size piece of the water-absorbent nylon nonwoven layer of Preparative Example B above to form a composite filter membrane, and the periphery of this composite filter membrane was sealed with an annular gasket die-cut from biaxially oriented polypropylene (BOPP, 1.6 mil (0.04mm) thickness) film coated with a thin layer of pressure sensitive adhesive

(96 wt.% iso-octyl acrylate and 4 wt.% acrylic acid), to complete formation of a composite filter body. The ability of the composite filter membrane to retain water was determined by weighing the dry composite filter membrane (dry weight = 323 milligrams), then filtering 50 milliliters of phosphate-buffered saline with the aid of a vacuum filtration apparatus, and then weighing the wet composite filter membrane (wet weight = 1530 milligrams; water weight = 1530 milligrams - 323 milligrams = 1207 milligrams).

Preparative Example D (Filter assembly)

An annular gasket was die-cut from biaxially-oriented polypropylene (BOPP, 1.6 mil (0.04 millimeter) thickness) film coated with a thin layer of pressure sensitive adhesive (96 wt.% iso-octyl acrylate and 4 wt.% acrylic acid). The outer diameter of the gasket was 52 millimeters and the inner diameter of the gasket was 45 millimeters.

A circular hole (44 millimeters in diameter) was die cut from a rectangular sheet (75 millimeters by 95 millimeters) of BOPP (1.6 mil (0.04 millimeter) thickness). A nylon membrane filter (47 millimeters, 0.45 millimeter nominal pore size, obtained from Alltech Associates (Deerfield, IL)) was centered over the hole on the adhesive-coated side of the BOPP sheet. The adhesive side of the gasket was centered over the outer edge of the membrane filter and was pressed against the filter and the BOPP sheet, sealing the edge of the membrane filter to the BOPP sheet. Next, another adhesive gasket (of the same dimensions) was die-cut to attach a water-absorbent nylon nonwoven layer of Preparative Example C, of equal diameter to the membrane, to the opposite side of the BOPP film.

Example 1 - Detection Article

A 7/8 inch (22 millimeter) diameter circular hole was die-cut into a 8 x 10 cm (3 inch x 4 inch) piece of biaxially-oriented polypropylene (BOPP, 1.6 mil (0.04 millimeter) thickness) film (obtained from Vifan Inc, Morristown, TN). A larger 50 millimeter circular hole was die-cut into a 8 x 10 cm (3 inch x 4 inch) piece of foam dam (0.56 millimeters thickness) and the foam dam was then adhered to the previously die-cut BOPP film using the adhesive present on the foam dam. Next, adhesive gaskets were die-cut out of adhesive coated BOPP to have an outside diameter of approximately 1 3/16 inches (30 millimeters) and an inside diameter of approximately 3/4 inch (19 millimeters) and were used to attach a 25 millimeter diameter 0.45 micrometer pore size membrane obtained from Whatman Inc. (Florham Park, NJ) under the trade designation "WHATMAN POLYCARBONATE MEMBRANE FILTER NO. 111107" to the opposite side of the BOPP film, overlaying the die-cut hole. Next, another adhesive gasket (of the same dimensions) was die-cut to attach a water-absorbent nylon nonwoven layer of Preparative Example C, of equal diameter to the membrane, to the opposite side of the BOPP film, to complete formation of a filter assembly.

The filter assembly so prepared was inserted between the cover sheet and base member of an aerobic count plate obtained from 3M Company (St. Paul. MN) under the trade designation "AC PETRIFILM", where the cover sheet had been removed and powder coated with Standard Methods Nutrients. The powder coating was performed by placing the cover sheet into a plastic bag containing 5 grams of Standard Methods Nutrients and shaking the bag for 30 seconds. The filter assembly was attached to the base member of the "AC PETRIFILM" using double-sided hinge tape along an edge, and the powder coated cover sheet was then similarly attached to the exposed side of the filtration sheet along the same edge.

The scope of the present invention should not be limited to the specific illustrative structures described herein, but is rather defined by the attached claims.

**Claims**

**1.** A filter plate article for testing the presence of microorganisms in an aqueous sample comprising:

a base member comprising a self-supporting water impervious substrate with first and second generally opposed major surfaces;
a filter assembly defining a filter assembly aperture therein, and having a composite filter body mounted across the filter assembly aperture; wherein the composite filter body comprises:

- a microporous membrane having pores with a nominal range of 0.05 μm to 1.2 μm, measured according to the description, and
- a water-absorbent layer; and

a cover sheet;
wherein the filter assembly is positioned between the cover sheet and the base member;

wherein the filter assembly is attached to the base member, and wherein the cover sheet is attached directly or indirectly to the base member; and
wherein the cover sheet or the base member comprises a dry coating that includes a water-soluble gelling agent, **characterized in that**

the water-absorbent layer is in fluid communication with the microporous membrane and the water-absorbent layer is positioned between the microporous membrane and the base member,
the water-absorbent layer absorbs a weight of water that is at least 0.5 times the weight of the water-absorbent layer prior to absorbing water and wherein the water-absorbent layer comprises a fibrous substrate that is functionalized with a grafted hydrogel polymer, and
the grafted hydrogel polymer includes grafting monomer units grafted to the surface of the fibrous substrate and wherein the grafting monomer units are selected from the group consisting of ionic grafting monomer units, non-ionic hydrophilic grafting monomer units, and mixtures thereof.

**2.** The article of claim 1, further comprising a spacer layer defining a spacer aperture therein, wherein the spacer layer is mounted on a major face of the filter assembly facing the first major surface of the base member, and wherein the spacer aperture is in fluid communication with the filter assembly aperture.

**3.** The article of any preceding claim, wherein the dry coating of the cover sheet or base member comprises a nutrient medium.

**4.** The article of any preceding claim, wherein the dry coating of the cover sheet or base member comprises a detection reagent.

**5.** The article of any preceding claim, wherein the microporous membrane comprises a material selected from the group consisting of polyethersulfone, nylon, polycarbonate, polyester, cellulose acetate, mixed cellulose esters, polyvinylidene fluoride, nitrocellulose, a ceramic, and any combination of these.

**6.** The article of any preceding claim, wherein the water-absorbent layer comprises a nonwoven substrate having:

an average fiber size in a range from 0.7 micrometer to 15 micrometers; and
a void volume in a range from 50% to 95%, both measured according to the description.

**7.** The article of claim 6, wherein the average fiber size is in a range from 1 micrometer to 6 micrometers.

**8.** The article of claim 6, wherein the ionic grafting monomer units comprise cationic monomer units, anionic monomer units, or a combination thereof.

**9.** The article of claim 6, wherein the non-ionic hydrophilic monomer units are selected from the group consisting of dimethylacrylamide and 2-hydroxyethyl(meth)acrylate.

**10.** The article of claim 6, wherein a weight of the hydrogel polymer comprising the monomer units grafted to the surface of the nonwoven substrate is in a range from 0.5 to 5 times a weight of the nonwoven substrate.

**11.** The article of claim 6, wherein the water-absorbent nonwoven substrate is a hydrophilic thermoplastic polymer substrate, wherein the substrate is formed from a thermoplastic polymeric material selected from polyolefins, poly(isoprenes), poly(butadienes), fluorinated polymers, chlorinated, polymers, polyamides, polyimides, polyethers, poly( ether sulfones), poly(sulfones), poly(vinyl acetates), copolymers of vinyl acetate, such as poly(ethylene)-co-poly(vinyl alcohol), poly(phosphazenes), poly(vinyl esters), poly(vinyl ethers), poly(vinyl alcohols), and poly(carbonates).

**12.** The article of claim 11, wherein water-absorbent nonwoven substrate is a polyamide nonwoven substrate.

**13.** The article of claim 12, wherein the water-absorbent nonwoven substrate is a nylon nonwoven substrate having an average effective fiber diameter of not greater than 1 micrometer.

**14.** A method of testing for the presence of a microorganism in an aqueous sample, the method comprising:

providing the filter plate article of any one of claims 1 to 13;
providing an aqueous sample;
passing the aqueous sample firstly through the microporous membrane and secondly through the water-absorbent layer, wherein the water absorbent layer retains an aliquot of water from the aqueous sample;
incubating the filter plate article for an incubation period, wherein at least a portion of the aliquot of water contacts the microporous membrane throughout the incubation period; and
observing an indication of the presence or absence of microbial growth.

**15.** The method of claim 14, further comprising:

providing a filtration apparatus, the filtration apparatus comprising a filter seat comprising a filter support surface and defining a filter seat inlet and a filter seat outlet; and
placing the water-absorbent layer of the filter plate article against the filter support surface prior to passing the aqueous sample firstly through the microporous membrane and secondly through the water-absorbent layer.

**Patentansprüche**

**1.** Filterplattenartikel zum Testen des Vorhandenseins von Mikroorganismen in einer wässrigen Probe, umfassend:

ein Basiselement, das ein selbsttragendes wasserundurchlässiges Substrat mit einer ersten und einer zweiten, im Allgemeinen gegenüberliegenden Hauptfläche umfasst;
eine Filteranordnung, die eine Filteranordnungsöffnung darin definiert und einen zusammengesetzten Filterkörper aufweist, der über der Filteranordnungsöffnung angebracht ist; wobei der zusammengesetzte Filterkörper umfasst:

- eine mikroporöse Membran mit Poren in einem Nennbereich von 0,05 um bis 1,2 um, gemessen gemäß der Beschreibung, und
- eine wasserabsorbierende Schicht; und

eine Abdeckfolie;
wobei die Filteranordnung zwischen der Abdeckfolie und dem Basiselement angeordnet ist;
wobei die Filteranordnung an dem Basiselement befestigt ist und wobei die Abdeckfolie direkt oder indirekt an dem Basiselement befestigt ist; und
wobei die Abdeckfolie oder das Basiselement eine trockene Beschichtung umfasst, die ein wasserlösliches Geliermittel enthält,
**dadurch gekennzeichnet, dass**
die wasserabsorbierende Schicht in Fluidverbindung mit der mikroporösen Membran steht und die wasserabsorbierende Schicht zwischen der mikroporösen Membran und dem Basiselement angeordnet ist,
die wasserabsorbierende Schicht ein Gewicht an Wasser absorbiert, das mindestens das 0,5-Fache des Gewichts der wasserabsorbierenden Schicht vor der Absorption von Wasser beträgt, und wobei die wasserabsorbierende Schicht ein faseriges Substrat umfasst, das mit einem gepfropften Hydrogelpolymer funktionalisiert ist, und
das gepfropfte Hydrogelpolymer Pfropfmonomereinheiten enthält, die auf die Oberfläche des faserigen Substrats gepfropft sind, und wobei die Pfropfmonomereinheiten aus der Gruppe ausgewählt sind, die aus ionischen Pfropfmonomereinheiten, nichtionischen hydrophilen Pfropfmonomereinheiten und Mischungen davon besteht.

**2.** Artikel nach Anspruch 1, der ferner eine Abstandshalterschicht umfasst, die eine Abstandshalteröffnung darin definiert, wobei die Abstandshalterschicht an einer Hauptfläche der Filteranordnung angebracht ist, die der ersten Hauptfläche des Basiselements zugewandt ist, und wobei die Abstandshalteröffnung in Fluidverbindung mit der Öffnung der Filteranordnung steht.

**3.** Artikel nach einem der vorhergehenden Ansprüche, wobei die trockene Beschichtung der Abdeckfolie oder des Basiselements ein Nährmedium enthält.

**4.** Artikel nach einem der vorhergehenden Ansprüche, wobei die trockene Beschichtung der Abdeckfolie oder des Basiselements ein Nachweisreagenz enthält.

5. Artikel nach einem der vorhergehenden Ansprüche, wobei die mikroporöse Membran ein Material umfasst, das aus der Gruppe ausgewählt ist, die aus Polyethersulfon, Nylon, Polycarbonat, Polyester, Celluloseacetat, gemischten Celluloseestern, Polyvinylidenfluorid, Nitrocellulose, einer Keramik und einer beliebigen Kombination dieser Materialien besteht.

6. Artikel nach einem der vorhergehenden Ansprüche, wobei die wasserabsorbierende Schicht ein Vliesstoffsubstrat umfasst, das Folgendes aufweist:

 eine durchschnittliche Fasergröße in einem Bereich von 0,7 Mikrometern bis 15 Mikrometern; und
 ein Hohlraumvolumen im Bereich von 50 % bis 95 %, jeweils gemessen gemäß der Beschreibung.

7. Artikel nach Anspruch 6, wobei die durchschnittliche Fasergröße in einem Bereich von 1 Mikrometer bis 6 Mikrometern liegt.

8. Artikel nach Anspruch 6, wobei die ionischen Pfropfmonomereinheiten kationische Monomereinheiten, anionische Monomereinheiten oder eine Kombination davon umfassen.

9. Artikel nach Anspruch 6, wobei die nichtionischen hydrophilen Monomereinheiten aus der Gruppe ausgewählt sind, die aus Dimethylacrylamid und 2 - Hydroxyethyl(meth)acrylat besteht.

10. Artikel nach Anspruch 6, wobei ein Gewicht des Hydrogelpolymers, das die auf die Oberfläche des Vliesstoffsubstrats gepfropften Monomereinheiten umfasst, im Bereich des 0,5- bis 5-Fachen des Gewichts des Vliesstoffsubstrats liegt.

11. Artikel nach Anspruch 6, wobei das wasserabsorbierende Vliesstoffsubstrat ein hydrophiles thermoplastisches Polymersubstrat ist, wobei das Substrat aus einem thermoplastischen Polymermaterial gebildet ist, das aus Polyolefinen, Poly(isoprenen), Poly(butadienen), fluorierten Polymeren, chlorierten Polymeren, Polyamiden, Polyimiden, Polyethern, Poly(ethersulfonen), Poly(sulfonen), Poly(vinylacetaten), Copolymeren von Vinylacetat wie Poly(ethylen)-copoly(vinylalkohol), Poly(phosphazenen), Poly(vinylestern), Poly(vinylethern), Poly(vinylalkoholen) und Poly(carbonaten) gebildet ist.

12. Artikel nach Anspruch 11, wobei das wasserabsorbierende Vliesstoffsubstrat ein Polyamidvliesstoffsubstrat ist.

13. Artikel nach Anspruch 12, wobei das wasserabsorbierende Vliesstoffsubstrat ein Nylonvliesstoffsubstrat mit einem durchschnittlichen effektiven Faserdurchmesser von nicht mehr als 1 Mikrometer ist.

14. Verfahren zum Testen auf das Vorhandensein eines Mikroorganismus in einer wässrigen Probe, wobei das Verfahren umfasst:

 Bereitstellen des Filterplattenartikels nach einem der Ansprüche 1 bis 13;
 Bereitstellen einer wässrigen Probe;
 Durchleiten der wässrigen Probe erstens durch die mikroporöse Membran und zweitens durch die wasserabsorbierende Schicht, wobei die wasserabsorbierende Schicht ein Aliquot an Wasser aus der wässrigen Probe zurückhält;
 Inkubieren des Filterplattenartikels für eine Inkubationszeit, wobei mindestens ein Teil des Aliquots an Wasser die mikroporöse Membran während der gesamten Inkubationszeit berührt; und
 Beobachten eines Hinweises auf das Vorhandensein oder Nichtvorhandensein von mikrobiellem Wachstum.

15. Verfahren nach Anspruch 14, das ferner umfasst:

 Bereitstellen einer Filtrationsvorrichtung, wobei die Filtrationsvorrichtung einen Filtersitz umfasst, der eine Filterträgerfläche umfasst und einen Filtersitzeinlass und einen Filtersitzauslass definiert; und
 Anlegen der wasserabsorbierenden Schicht des Filterplattenartikels an die Filterträgeroberfläche, bevor die wässrige Probe erstens durch die mikroporöse Membran und zweitens durch die wasserabsorbierende Schicht geleitet wird.

## Revendications

1. Article sous forme de plaque filtrante conçu pour une analyse visant à déterminer la présence de microorganismes dans un échantillon aqueux, comprenant :

   un élément de base comprenant un substrat autoportant imperméable à l'eau comportant des première et deuxième surfaces principales généralement opposées ;
   un ensemble filtre délimitant une ouverture d'ensemble filtre à l'intérieur de celui-ci, et comportant un corps filtre composite monté en travers de l'ouverture de l'ensemble filtre ; le corps filtre composite comprenant :

   - une membrane microporeuse comportant des pores situés dans une plage nominale de 0,05 um à 1,2 um, mesurée conformément à la description, et
   - une couche absorbant l'eau ; et

   une feuille de couverture ;
   dans lequel l'ensemble filtre est positionné entre la feuille de couverture et l'élément de base ;
   dans lequel l'ensemble filtre est fixé à l'élément de base, et dans lequel la feuille de couverture est fixée directement ou indirectement à l'élément de base ; et
   dans lequel la feuille de couverture ou l'élément de base comprend un revêtement sec qui contient un gélifiant hydrosoluble,

   **caractérisé en ce que**

   la couche absorbant l'eau est en communication fluidique avec la membrane microporeuse et la couche absorbant l'eau est positionnée entre la membrane microporeuse et l'élément de base,
   la couche absorbant l'eau absorbe un poids d'eau qui est au moins 0,5 fois le poids de la couche absorbant l'eau avant l'absorption de l'eau et dans lequel la couche absorbant l'eau comprend un substrat fibreux qui est fonctionnalisé avec un polymère hydrogel greffé, et
   le polymère hydrogel greffé contient des unités monomères de greffage greffées à la surface du substrat fibreux et dans lequel les unités monomères de greffage sont sélectionnées dans le groupe constitué d'unités monomères de greffage ioniques, d'unités monomères de greffage hydrophiles non ioniques, et de mélanges de celles-ci.

2. Article selon la revendication 1, comprenant en outre une couche d'espacement délimitant une ouverture d'espacement à l'intérieur de celle-ci, dans lequel la couche d'espacement est montée sur une face principale de l'ensemble filtre faisant face à la première surface principale de l'élément de base, et dans lequel l'ouverture d'espacement est en communication fluidique avec l'ouverture de l'ensemble filtre.

3. Article selon l'une quelconque des revendications précédentes, dans lequel le revêtement sec de la feuille de couverture ou de l'élément de base comprend un milieu nutritif.

4. Article selon l'une quelconque des revendications précédentes, dans lequel le revêtement sec de la feuille de couverture ou de l'élément de base comprend un réactif de détection.

5. Article selon l'une quelconque des revendications précédentes, dans lequel la membrane microporeuse comprend un matériau sélectionné dans le groupe constitué de la polyéthersulfone, du nylon, du polycarbonate, du polyester, de l'acétate de cellulose, d'esters de cellulose mixtes, du fluorure de polyvinylidène, de la nitrocellulose, d'une céramique, et de n'importe quelle combinaison de ceux-ci.

6. Article selon l'une quelconque des revendications précédentes, dans lequel la couche absorbant l'eau comprend un substrat non tissé ayant :

   une taille moyenne de fibre dans une plage de 0,7 micromètre à 15 micromètres ; et
   un volume de vide dans une plage de 50 % à 95 %, tous deux mesurés conformément à la description.

7. Article selon la revendication 6, dans lequel la taille moyenne de fibre est dans une plage de 1 micromètre à 6 micromètres.

**8.** Article selon la revendication 6, dans lequel les unités monomères de greffage ioniques comprennent des unités monomères cationiques, des unités monomères anioniques, ou une combinaison de celles-ci.

**9.** Article selon la revendication 6, dans lequel les unités monomères hydrophiles non ioniques sont sélectionnées dans le groupe constitué du diméthylacrylamide et du 2-hydroxyéthyl(méth)acrylate.

**10.** Article selon la revendication 6, dans lequel un poids du polymère hydrogel comprenant les unités monomères greffées à la surface du substrat non tissé est dans une plage de 0,5 à 5 fois un poids du substrat non tissé.

**11.** Article selon la revendication 6, dans lequel le substrat non tissé absorbant l'eau est un substrat polymère thermoplastique hydrophile, dans lequel le substrat est formé d'un matériau polymère thermoplastique sélectionné parmi des polyoléfines, des poly(isoprènes), des poly(butadiènes), des polymères fluorés, des polymères chlorés, des polyamides, des polyimides, des polyéthers, des poly(éthersulfones), des poly(sulfones), des poly(acétates de vinyle), des copolymères d'acétate de vinyle, tels que le poly(éthylène)-co-poly(alcool vinylique), des poly(phosphazènes), des poly(esters vinyliques), des poly(éthers vinyliques), des poly(alcools vinyliques), et des poly(carbonates).

**12.** Article selon la revendication 11, dans lequel le substrat non tissé absorbant l'eau est un substrat non tissé en polyamide.

**13.** Article selon la revendication 12, dans lequel le substrat non tissé absorbant l'eau est un substrat non tissé en nylon ayant un diamètre de fibre effectif moyen ne dépassant pas 1 micromètre.

**14.** Procédé d'analyse visant à déterminer la présence d'un micro-organisme dans un échantillon aqueux, le procédé comprenant :

la fourniture de l'article sous forme de plaque filtrante selon l'une quelconque des revendications 1 à 13 ;
la fourniture d'un échantillon aqueux ;
le fait de faire passer l'échantillon aqueux d'abord à travers la membrane microporeuse et ensuite à travers la couche absorbant l'eau, la couche absorbant l'eau retenant une aliquote d'eau à partir de l'échantillon aqueux ;
l'incubation de l'article sous forme de plaque filtrante pendant une période d'incubation, au moins une partie de l'aliquote d'eau se trouvant en contact avec la membrane microporeuse pendant toute la période d'incubation ; et
l'observation d'une indication de la présence ou de l'absence de croissance microbienne.

**15.** Procédé selon la revendication 14, comprenant en outre :

la fourniture d'un appareil de filtration, l'appareil de filtration comprenant un siège de filtre comprenant une surface de support de filtre et délimitant une entrée de siège de filtre et une sortie de siège de filtre ; et
la mise en place de la couche absorbant l'eau de l'article sous forme de plaque filtrante contre la surface de support de filtre avant de faire passer l'échantillon aqueux d'abord à travers la membrane microporeuse, puis à travers la couche absorbant l'eau.

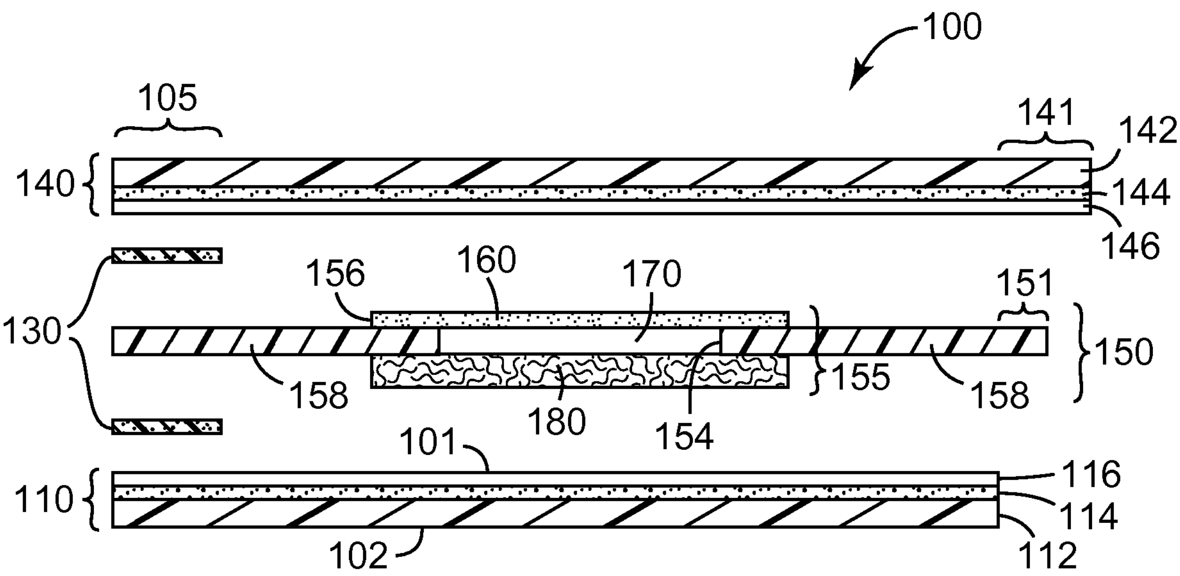
100
105
141
142
140
144
146
156
160
170
151
130
150
158
155
158
180
154
101
116
110
114
102
112

FIG. 1A

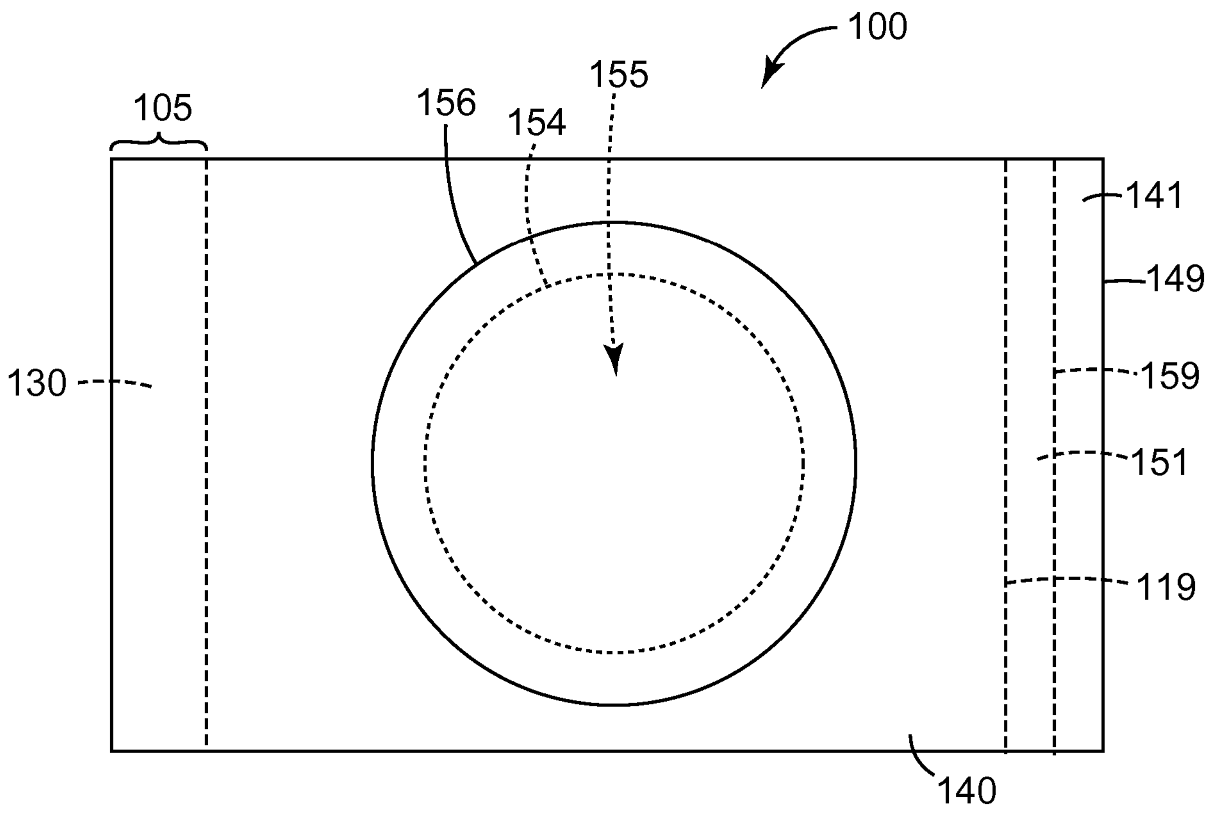
100
155
105
156
154
141
149
130
159
151
119
140

FIG. 1B

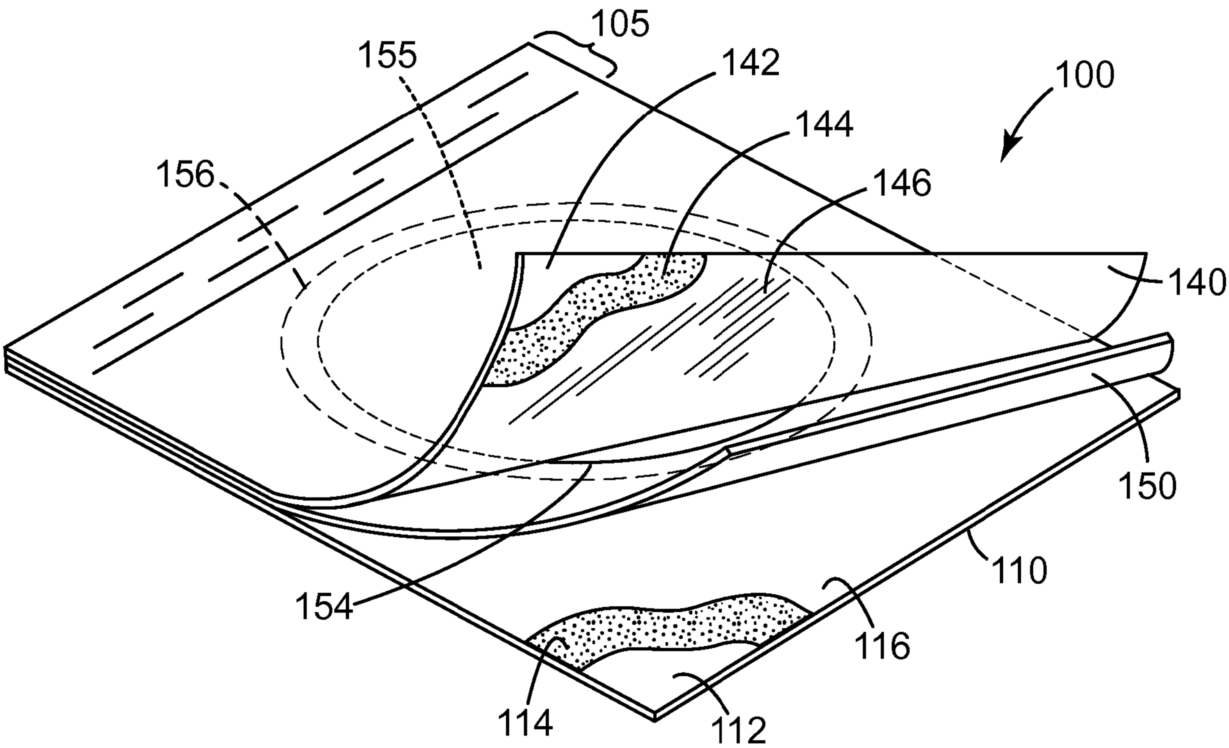


*FIG. 2*

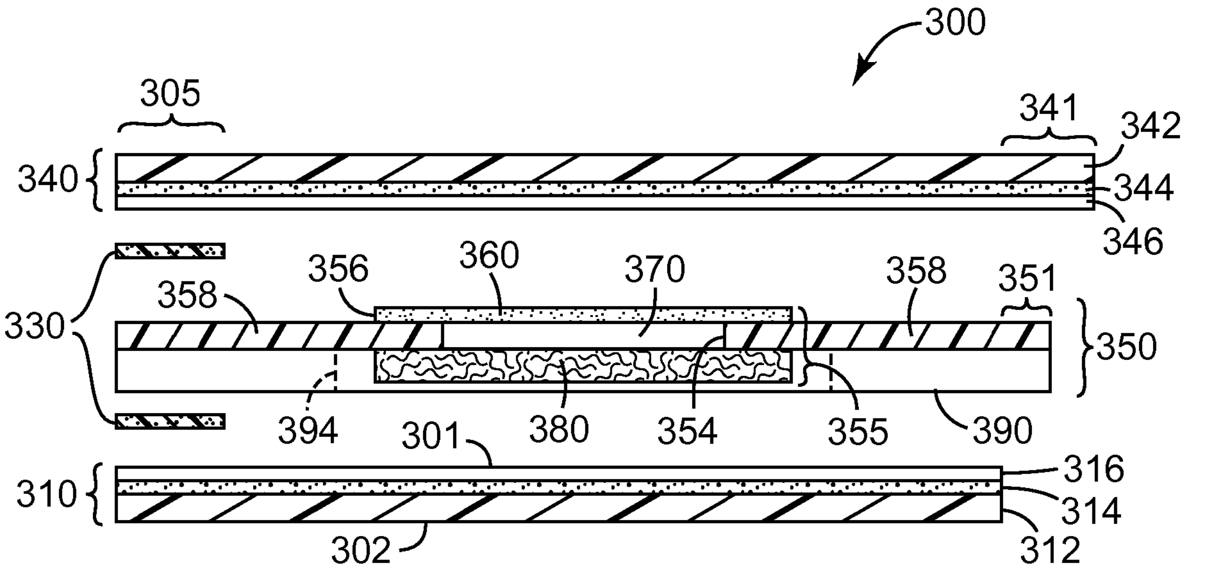
300
305
341
342
340
344
346
330
356
360
370
358
358
351
350
394
380
354
355
390
301
316
310
314
302
312

*FIG. 3A*

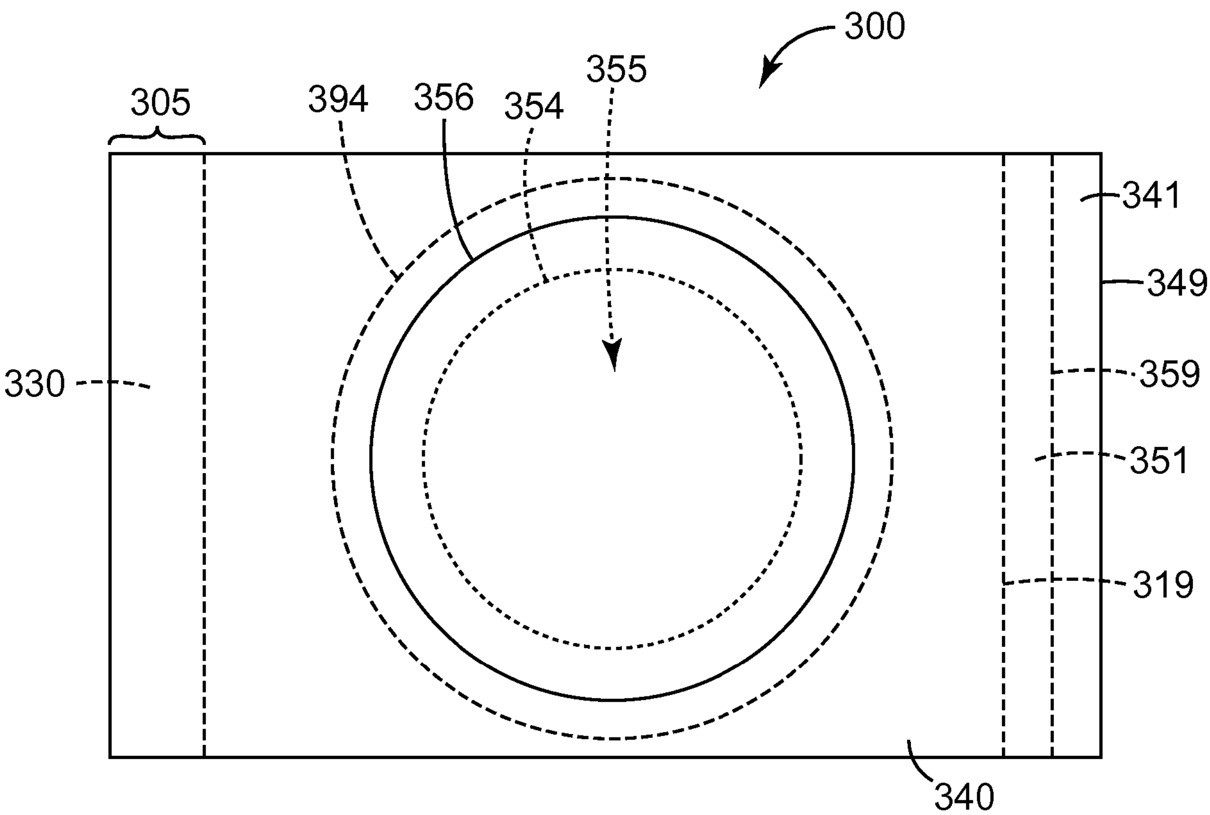
300
355
305
394
356
354
341
349
330
359
351
319
340

*FIG. 3B*

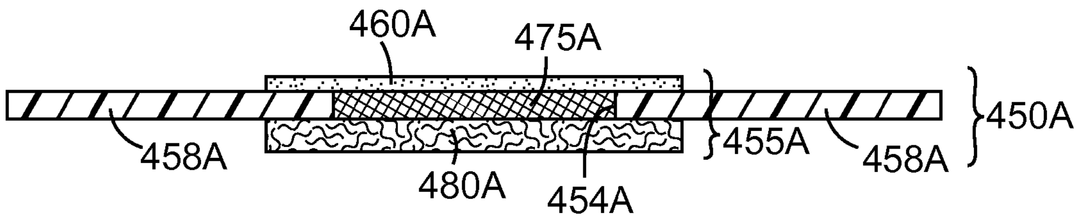


FIG. 4A

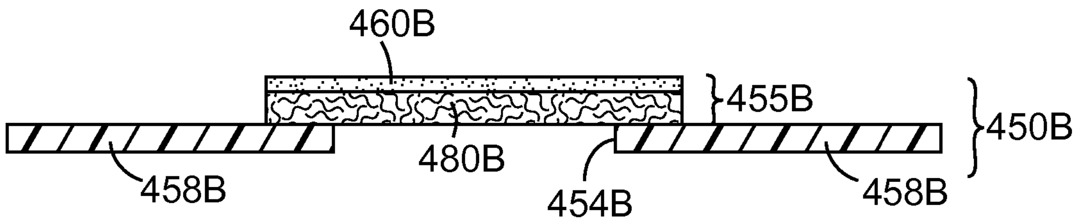


FIG. 4B

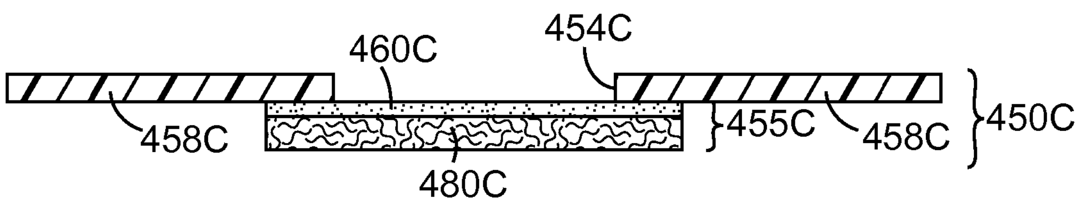


FIG. 4C

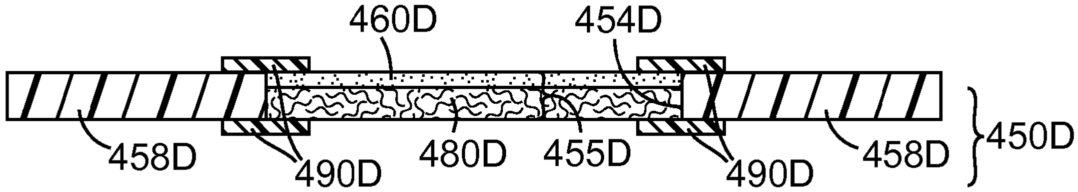


FIG. 4D

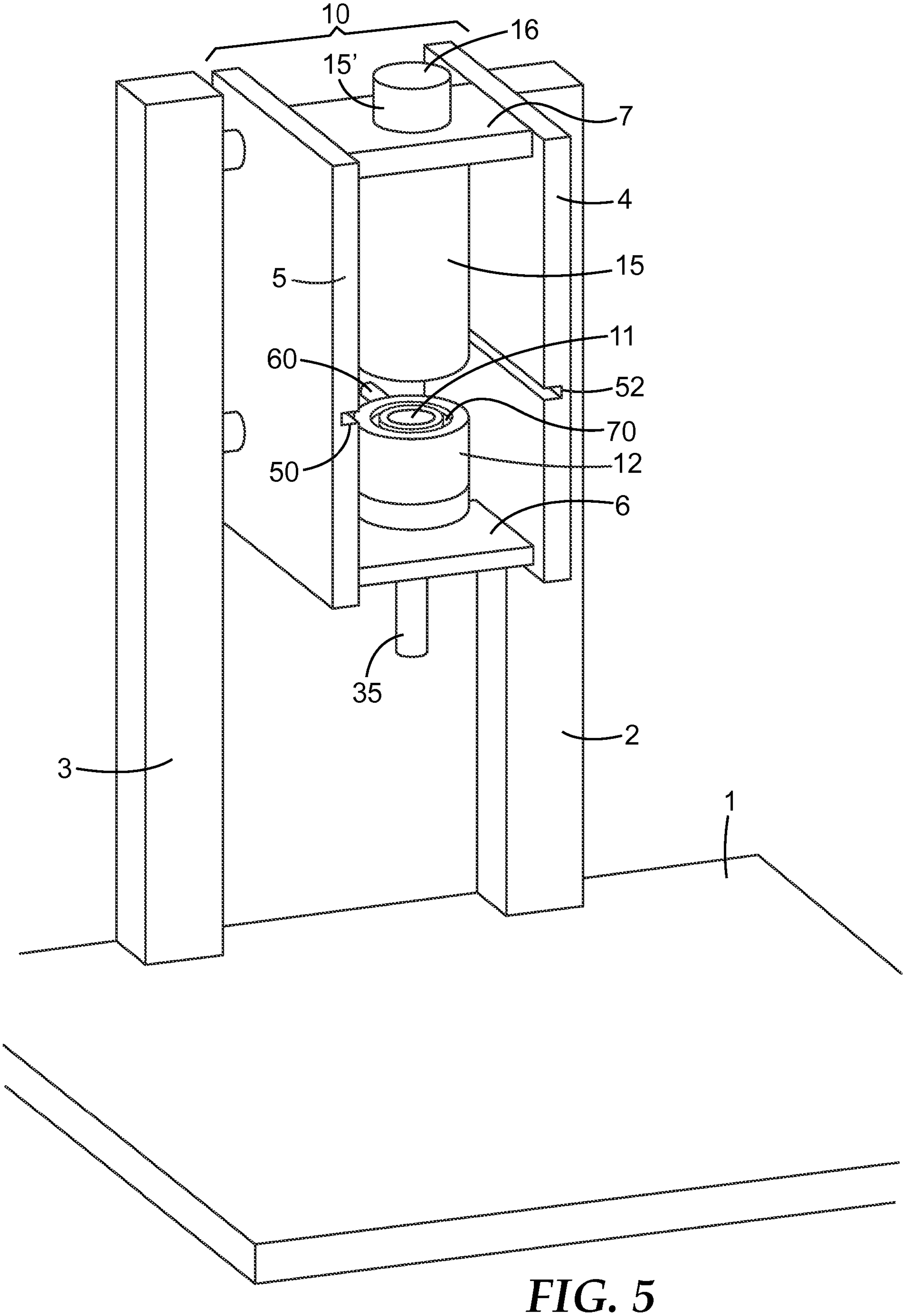


FIG. 5

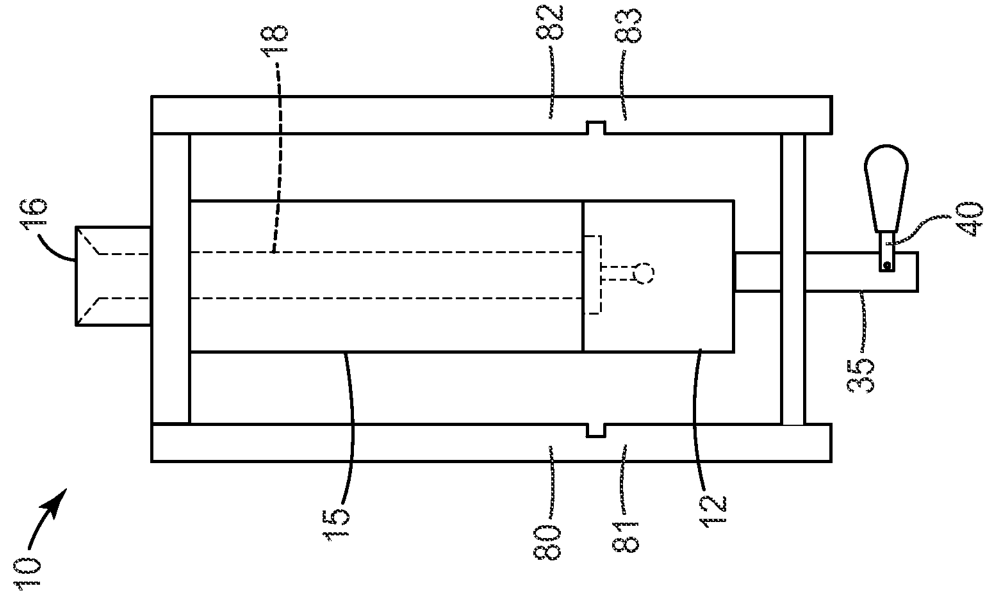
10
16
18
82
83
15
80
81
12
35
40

FIG. 6B

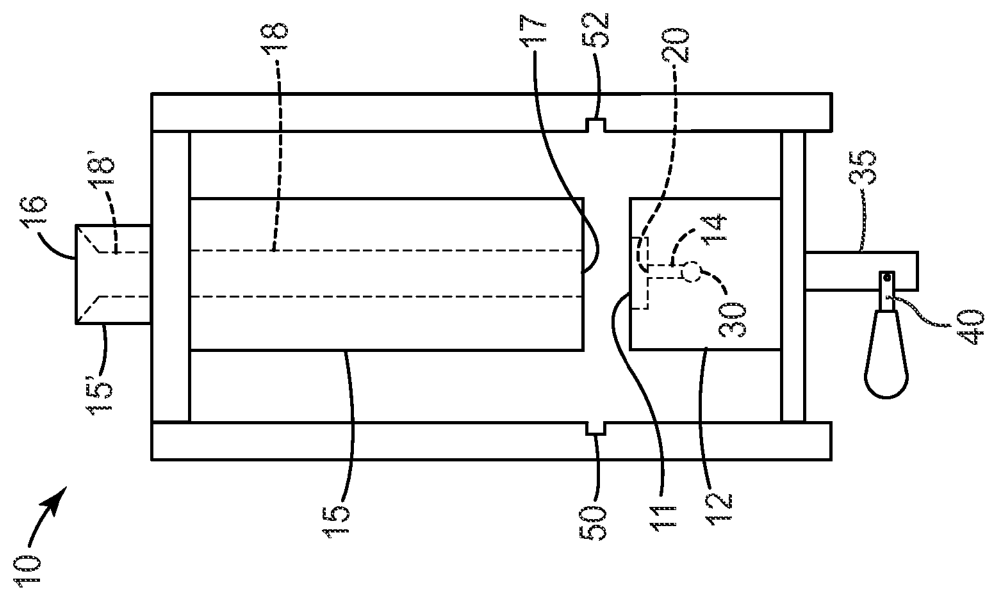
10
16
18'
18
17
52
20
15'
15
50
11
12
14
30
35
40

FIG. 6A

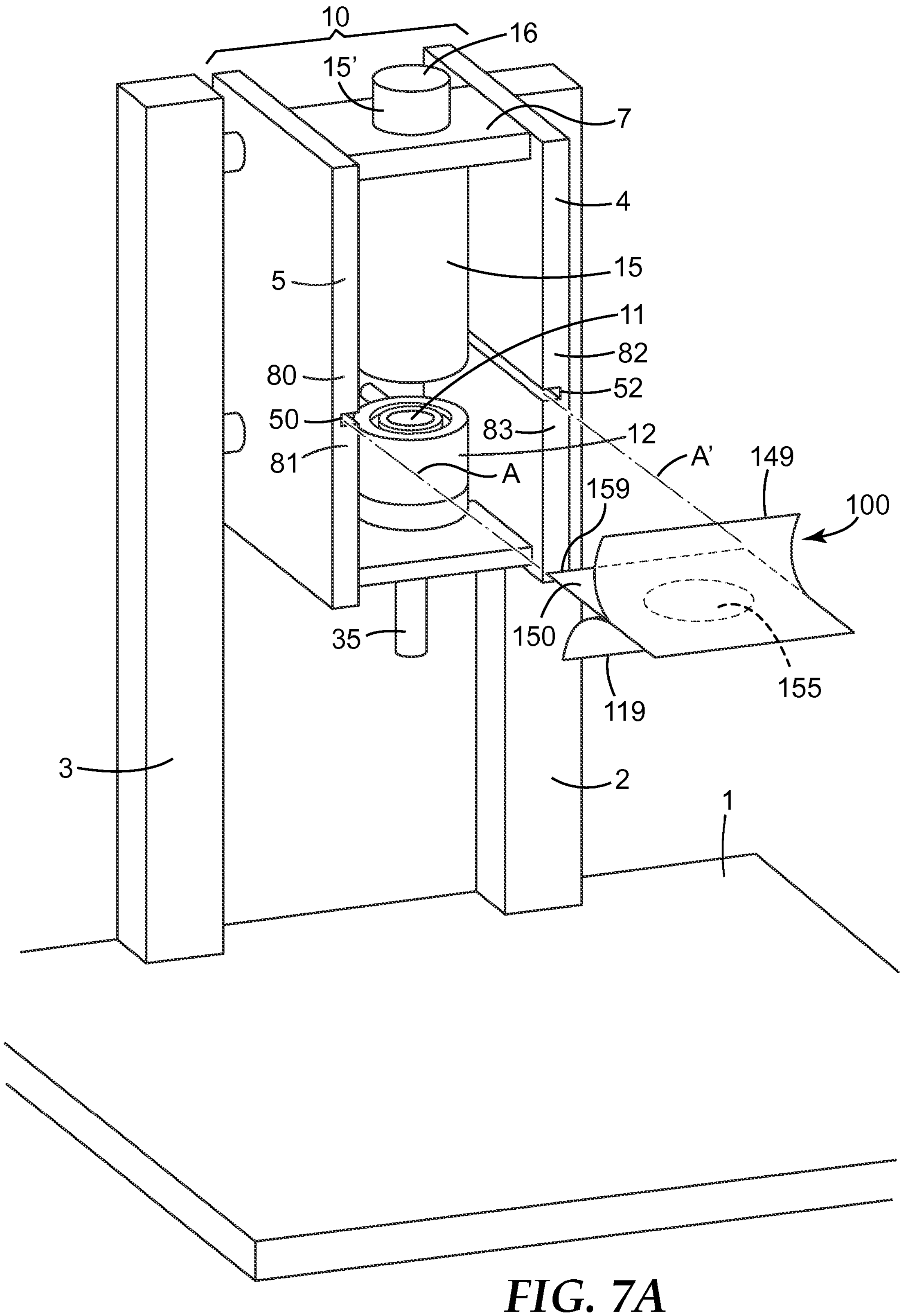
10
16
15'
7
4
5
15
11
82
80
52
50
83
12
81
A
A'
149
159
100
150
35
155
119
3
2
1

FIG. 7A

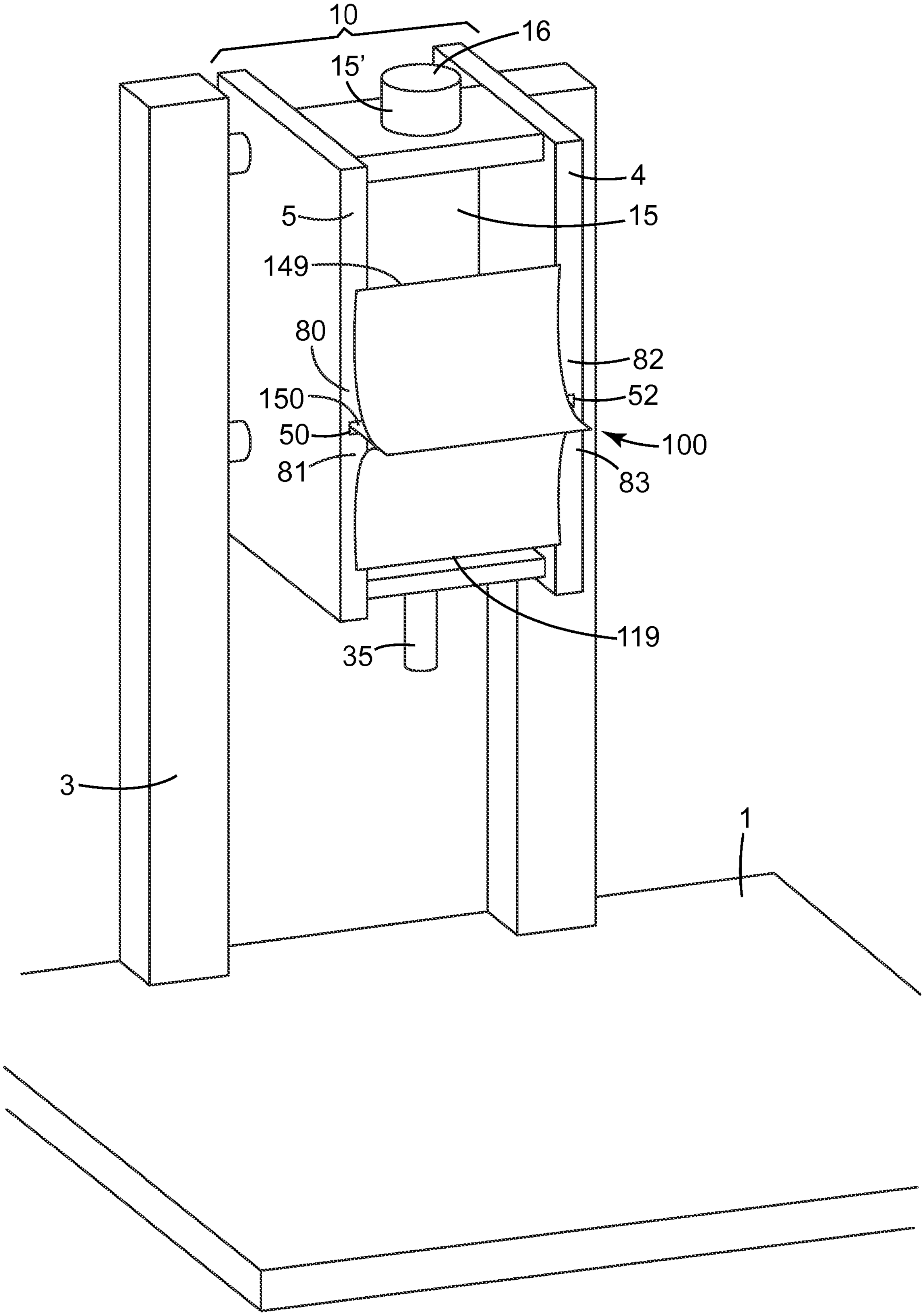


FIG. 7B

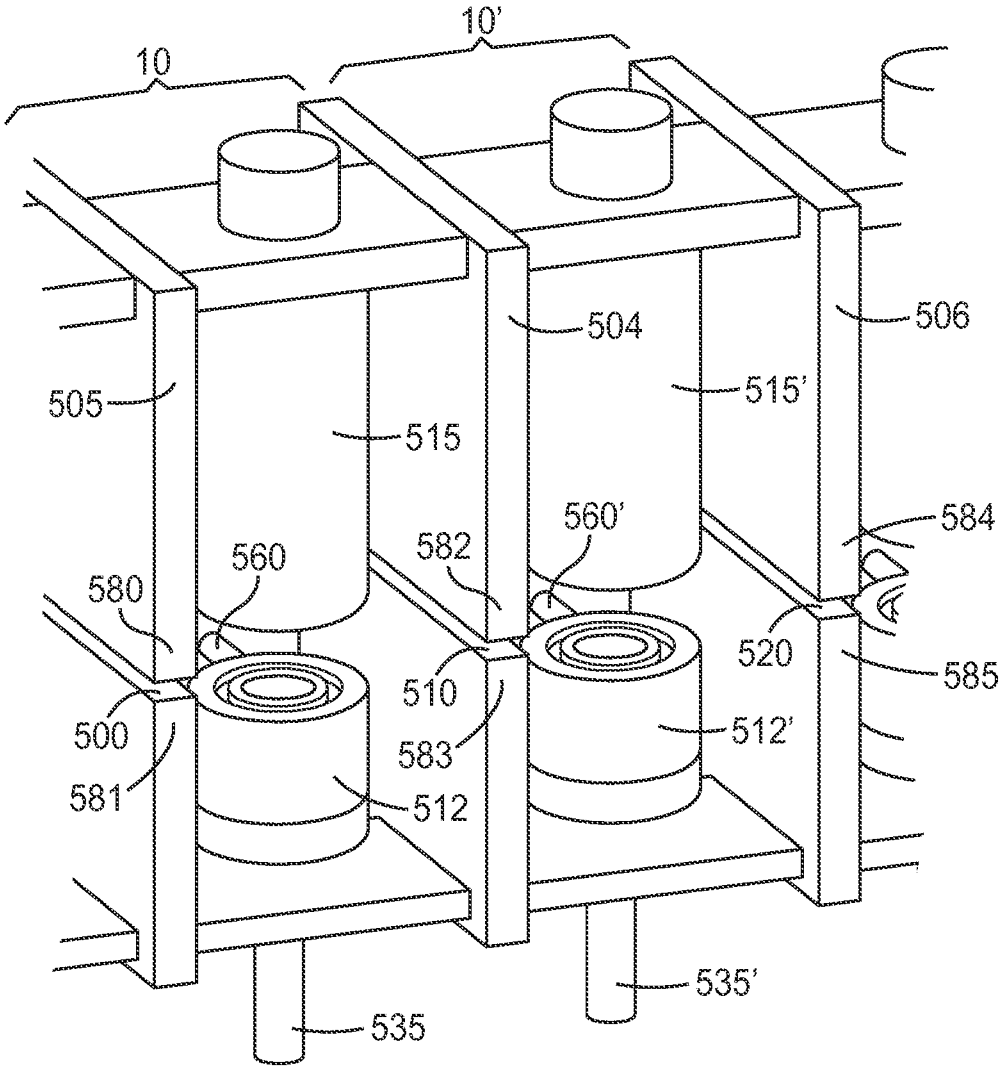
10
10'
505
515
504
515'
506
560
580
582
560'
584
520
585
510
500
583
512'
581
512
535
535'

*FIG. 8*

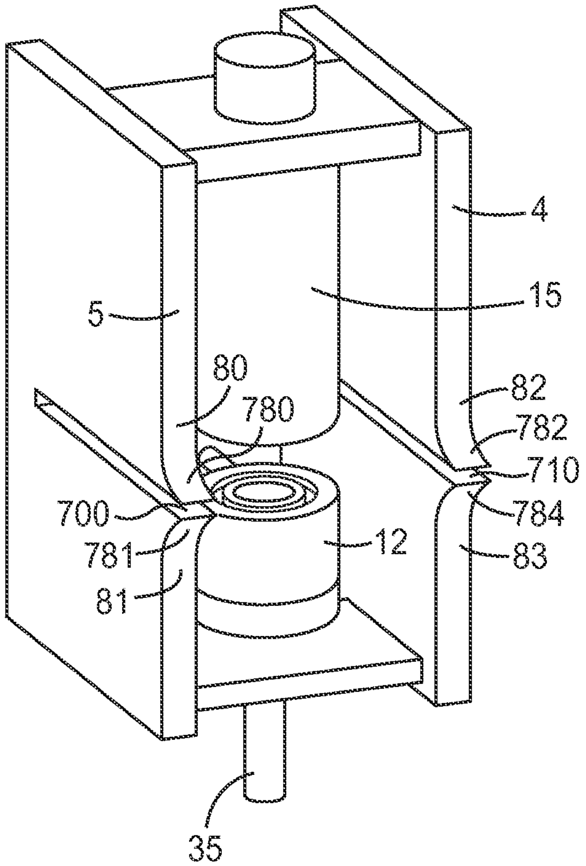
4
15
5
80
780
82
782
710
700
784
781
12
83
81
35

*FIG. 9*

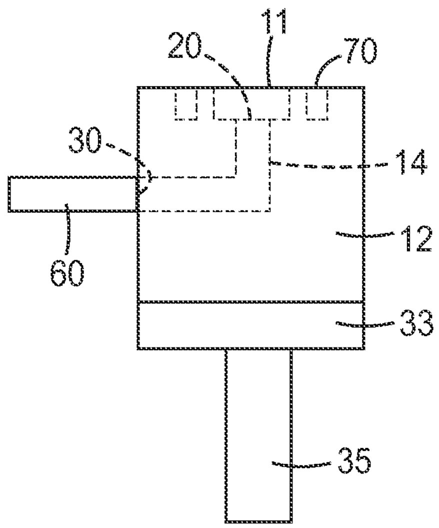
20
11
70
30
14
12
60
33
35

*FIG. 10*

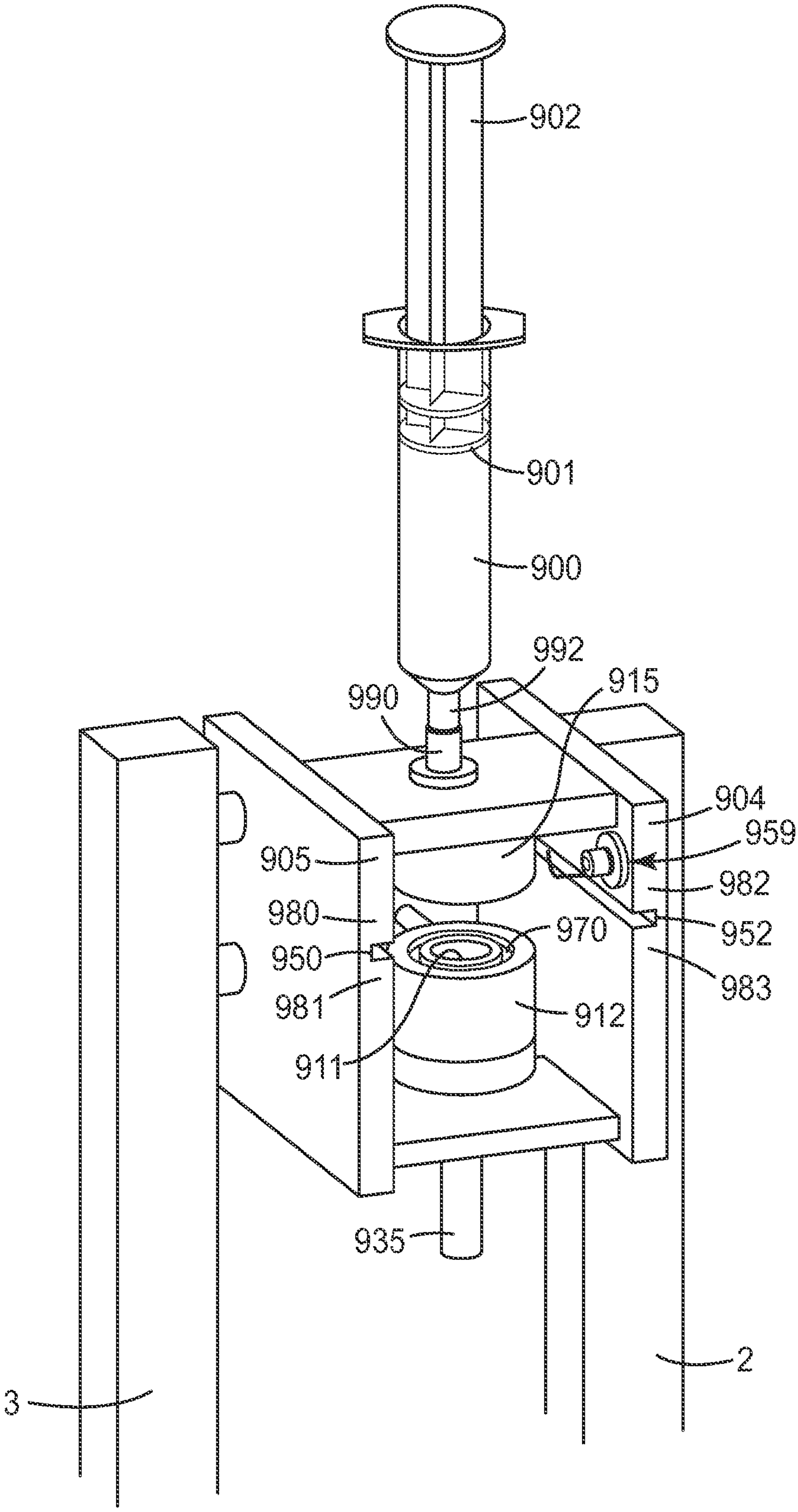
902
901
900
992
915
990
904
959
905
982
980
970
952
950
983
981
912
911
935
2
3

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5681712 A
- JP 9206062 A
- WO 2010077619 A1
- US 5348861 A
- US 7534600 B2
- US 2001017280 A1
- WO 2010022111 A1
- US 2010155323 A1
- US 4565783 A, Hansen
- US 5089413 A, Nelson
- US 5364766 A, Mach
- US 5443963 A, Lund
- US 5462860 A, Mach
- US 5601998 A, Mach
- US 5635367 A, Lund
- US 6413070 B, Meyering
- US 6513666 B, Meyering
- US 6776940 B, Meyering
- US 6056529 A, Meyering
- US 6264044 B, Meyering
- US 5006247 A, Dennison
- US 3876738 A, Marinaccio
- US 4707265 A, Barnes
- US 4473474 A, Ostreicher
- US 20100261801 A, Weiss
- US 4118531 A, Hauser
- US 7170739 B, Arora
- US 7112389 B, Arora
- US 7235122 B, Bryner
- US 20040116026 A
- US 39399 A, Allen
- US 3849241 A, Butin
- US 7374416 B, Cook
- US 4936934 A, Buehning
- US 6230776 B, Choi
- US 2010038488 W, Waller
- US 4157418 A, Heilmann
- US 20100155323 A, Weiss
- US 20070154651 A, Weiss
- US 20100075560 A, Seshadri
- US 61360489, Miller
- WO 2005024047 A, Graessle
- US 20040101954, Graessle
- US 20040102903 A, Graessle
- US 61187107


### Non-patent literature cited in the description


- Standard Methods for the Examination of Dairy Products. American Public Health Association
- **SADLER et al.** *J. Am Chem. Soc.,* 1956, vol. 78, 1251-1255
- **DAVIES, C. N.** The Separation of Airborne Dust and Particles. *Institution of Mechanical Engineers, London, Proceedings 1B,* 1952
- **WENTE.** Superfine Thermoplastic Fibers. *Indus. Eng. Chem,* 1956, vol. 48, 1342
- **WENTE et al.** Manufacture Of Superfine Organic Fibers. *Naval Research Laboratories Report,* 1954, (4364